# EUROPEAN PATENT SPECIFICATION

(11) **EP 3 918 331 B1**
(45) Date of publication and mention of the grant of the patent: **11.06.2025**
(21) Application number: 20708870.9
(22) Date of filing: 29.01.2020
(51) Int. Cl.: G01N 33/569

(54) **METHODS AND SYSTEMS FOR THE RAPID DETECTION OF LISTERIA USING INFECTIOUS AGENTS**
VERFAHREN UND SYSTEME ZUM SCHNELLEN NACHWEIS VON LISTERIEN MITHILFE VON INFEKTIONSERREGERN
PROCÉDÉS ET SYSTÈMES POUR LA DÉTECTION RAPIDE DE LISTERIA À L'AIDE D'AGENTS INFECTIEUX

(30) Priority: 29.01.2019 US 201962798248 P
(43) Date of publication of application: 08.12.2021
(73) Proprietor: Laboratory Corporation of America Holdings, Burlington, North Carolina 27215 (US)
(72) Inventor: GIL, Jose S., Winnetka, California 91306 (US); NGUYEN, Minh Mindy Bao, Shoreview, Minnesota 55126 (US); ANDERSON, Dwight Lyman, Minneapolis, Minnesota 55406 (US); ERICKSON, Stephen, White Bear Township, Minnesota 55110 (US)
(74) Representative: Forresters IP LLP
(86) International application number: PCT/US2020/015737
(87) International publication number: WO 2020/160190

(56) References cited:
- WO-A1-2019/173838
- STEVEN HAGENS ET AL: "Reporter bacteriophage A511:: celB transduces a hyperthermostable glycosidase from Pyrococcus furiosus for rapid and simple detection of viable Listeria cells", BACTERIOPHAGE, vol. 1, no. 3, 1 May 2011 (2011-05-01), pages 143 - 151, XP055192917, DOI: 10.4161/bact.1.3.16710
- LOESSNER M J ET AL: "Evaluation of luciferase reporter bacteriophage A511:luxAB for detection of listeria monocytogenes in contaminated foods", APPLIED AND ENVIRONMENTAL MICROBIOLOGY, AMERICAN SOCIETY FOR MICROBIOLOGY, US, vol. 63, no. 8, 1 August 1997 (1997-08-01), pages 2961 - 2965, XP002966690, ISSN: 0099-2240
- LOESSNER M J ET AL: "Construction of luciferase reporter bacteriophage A511::luxAB for rapid and sensitive detection of viable Listeria cells", APPLIED AND ENVIRONMENTAL MICROBIOLOGY, AMERICAN SOCIETY FOR MICROBIOLOGY, US, vol. 62, no. 4, 1 April 1996 (1996-04-01), pages 1133 - 1140, XP002079442, ISSN: 0099-2240
- LOESSNER M J ET AL: "ORGANIZATION AND TRANSCRIPTIONAL ANALYSIS OF THE LISTERIA PHAGE A511 LATE GENE REGION COMPRISING THE MAJOR CAPSID AND TAIL SHEATH PROTEIN GENES CPS AND TSH", JOURNAL OF BACTERIOLOGY,, vol. 177, no. 22, 1 November 1995 (1995-11-01), pages 6601 - 6609, XP000576355, ISSN: 0021-9193
- AKHTAR MASTURA ET AL: "Isolation, characterization and evaluation of virulent bacteriophages againstListeria monocytogenes", FOOD CONTROL, BUTTERWORTH, LONDON, GB, vol. 75, 24 December 2016 (2016-12-24), pages 108 - 115, XP029884221, ISSN: 0956-7135, DOI: 10.1016/J.FOODCONT.2016.12.035
- FAROOQ UMER ET AL: "Bacterial biosensing: Recent advances in phage-based bioassays and biosensors", BIOSENSORS AND BIOELECTRONICS, ELSEVIER SCIENCE LTD. UK, AMSTERDAM, NL, vol. 118, 30 July 2018 (2018-07-30), pages 204 - 216, XP085444763, ISSN: 0956-5663, DOI: 10.1016/J.BIOS.2018.07.058

## Description

### FIELD OF THE INVENTION

This invention relates to compositions, methods, systems, and kits for the detection of microorganisms using infectious agents.

### BACKGROUND

There is a strong interest in improving speed and sensitivity for detection of bacteria, viruses, and other microorganisms in biological, food, water, and clinical samples. Microbial pathogens can cause substantial morbidity among humans and domestic animals, as well as immense economic loss. Also, detection of microorganisms is a high priority for the Food and Drug Administration (FDA) and Centers for Disease Control (CDC), as well as the United States Department of Agriculture (USDA), given outbreaks of life-threatening or fatal illness caused by ingestion of food contaminated with certain microorganisms, e.g., *Listeria spp.*, *Salmonella spp.*, or *Staphylococcus spp.*

In particular, *Listeria spp.* are known to cause the potentially serious infection, listeriosis. *Listeria spp.,* such as *L. monocytogenes,* are typically transmitted through ingestion of contaminated food products. *L. monocytogenes* is a gram-positive bacterium commonly associated with contamination of food products, including but not limited to, milk, seafood, poultry, and meat. Food-borne illnesses, such as listeriosis, can be prevented by detecting contaminated foods prior to consumer availability.

Traditional microbiological tests for the detection of bacteria rely on non-selective and selective enrichment cultures followed by plating on selective media and further testing to confirm suspect colonies. Such procedures can require as long as seven days. For examples, traditional tests for the detection of *Listeria spp.* in food products are complex and time consuming requiring 24-48 hour enrichment periods followed by additional lengthy testing with a total time for detection ranging from 5-7 days. A variety of rapid methods have been investigated and introduced into practice to reduce the time requirement. However, these methods have drawbacks. For example, polymerase chain reaction (PCR) tests, which also include an amplification step and therefore are capable of both very high sensitivity and selectivity; are economically limited to a small sample size. With dilute bacterial suspensions, most small subsamples will be free of cells and therefore purification and/or lengthy enrichment steps are still required.

The time required for traditional biological enrichment is dictated by the growth rate of the target bacterial population of the sample, by the effect of the sample matrix, and by the required sensitivity. Due to the time required for cultivation, these methods can take up to eight days, depending upon the organism to be identified and the source of the sample. This lag time is generally unsuitable as the contaminated food, water, or other product may have already made its way into livestock or humans. In addition, increases in antibiotic-resistant bacteria and biodefense considerations make rapid identification of bacterial pathogens in water, food and clinical samples critical priorities worldwide.

Therefore, there is a need for more rapid, simple, and sensitive detection and identification of microorganisms, such as bacteria and other potentially pathogenic microorganisms.
Hagens et al., BACTERIOPHAGE, (2011), vol. 1, no. 3, pages 143 - 151, describes a reporter bacteriophage A511: :celB for detection of viable Listeria cells. Loessner et al., APPLIED AND ENVIRONMENTAL MICROBIOLOGY, AMERICAN SOCIETY FOR MICROBIOLOGY, US, (1997), vol. 63, no. 8, pages 2961 - 2965, describes a luciferase reporter bacteriophage A511: :luxAB for detection of Listeria monocytogenes in contaminated foods. Loessner et al., APPLIED AND ENVIRONMENTAL MICROBIOLOGY, AMERICAN SOCIETY FOR MICROBIOLOGY, US, (1996), vol. 62, no. 4, pages 1133 - 1140, describes the construction of the recombinant bacteriophage A511::luxAB for detection of Listeria cells. Loessner et al., BACTERIOLOGY,, (1995), vol. 177, no. 22, pages 6601 - 6609, looks at the organization and transcription of the Listeria phage A511 late gene region. Akhtar et al., FOOD CONTROL (2016), vol. 75, pages 108 - 115, describe the isolation, characterization and evaluation of virulent bacteriophages against *Listeria monocytogenes.* Farooq et al., BIOSENSORS AND BIOELECTRONICS, (2018), vol. 118, pages 204 - 216, describes the use of phage-based bioassays and biosensors.

### SUMMARY

Embodiments of the invention comprise compositions, methods, systems, and kits for the detection of *Listeria spp.* The invention may be embodied in a variety of ways.

In some aspects, the invention comprises a cocktail composition comprising at least one recombinant bacteriophage constructed from LMA4, comprising an indicator gene, and an exogenous promoter controlling transcription of the indicator gene, inserted into a late gene region of a bacteriophage genome, wherein the indicator gene is not contiguous with a gene encoding a structural bacteriophage protein and does not yield a fusion protein, and wherein expression of the indicator gene results in an indicator protein product, and wherein the recombinant bacteriophage specifically infects *Listeria spp..*

Disclosed are compositions that include a recombinant indicator bacteriophage as described herein. For example, compositions can include one or more wild-type or genetically modified infectious agents (e.g., bacteriophages) and one or more indicator genes. In some aspects, the compositions, methods, systems and kits may comprise a cocktail of at least one recombinant bacteriophage for use in detection of microorganisms such as *Listeria spp.*

In some aspects of methods for preparing recombinant indicator bacteriophage, the wild-type bacteriophage is a *Listeria spp.*-specific bacteriophage and the target pathogenic bacterium is *Listeria spp.* In some aspects, isolating a particular clone of recombinant bacteriophage comprises a limiting dilution assay for isolating a clone that demonstrates expression of the indicator gene.

Other aspects of the invention include methods for detecting *Listeria spp.* in a sample, including steps of incubating the sample with the cocktail composition described above, and detecting the indicator protein product produced by the recombinant bacteriophage, wherein positive detection of the indicator protein product indicates that *Listeria spp.* is present in the sample. The sample can be a food or water sample. In some embodiments, samples include environmental samples (e.g., sponges and swabs of surfaces or equipment for bacterial monitoring in factories and other processing facilities), or commercial samples. In some embodiments, the food sample comprises meat, fish, vegetables, eggs, dairy products, dried food products, or powdered infant formula.

In some embodiments of methods for detecting bacteria, the sample is first incubated in conditions favoring growth for an enrichment period of 24 hours or less, 23 hours or less, 22 hours or less, 21 hours or less, 20 hours or less, 19 hours or less, 18 hours or less, 17 hours or less, 16 hours or less, 15 hours or less, 14 hours or less, 13 hours or less, 12 hours or less, 11 hours or less, 10 hours or less, or 9 hours or less, 8 hours or less, 7 hours or less, 6 hours or less, 5 hours or less, 4 hours or less, 3 hours or less, or 2 hours or less. In some embodiments, the sample is not enriched prior to detection. In some embodiments, the total time to results is less than 28 hours, less than 27 hours, less than 26 hours, 25 hours, 24 hours, 23 hours, 22 hours, 21 hours, 20 hours, 19 hours, 18 hours, 17 hours, 16 hours, 15 hours, 14 hours, 13 hours, 12 hours, 11 hours, 10 hours, 9 hours, 8 hours, 7 hours, 6 hours, 5 hours, 4 hours, 3 hours or 2 hours. In some embodiments, the ratio of signal to background generated by detecting the indicator is at least 2.0 or at least 2.5 or at least 3.0. In some embodiments, the method detects as few as 1, 10, 15, 20, 30, 40, 50, 60, 70, 80, 90, or 100 of the specific bacteria in a sample of a standard size for the food safety industry.

Additional embodiments include systems and kits for detecting *Listeria spp.,* wherein the systems or kits comprise the cocktail composition described above, as defined in the appended claims. Some embodiments further include a substrate for reacting with an indicator to detect the soluble protein product expressed by the recombinant bacteriophage.

### BRIEF DESCRIPTION OF THE FIGURES

The present invention may be better understood by referring to the following nonlimiting figures.

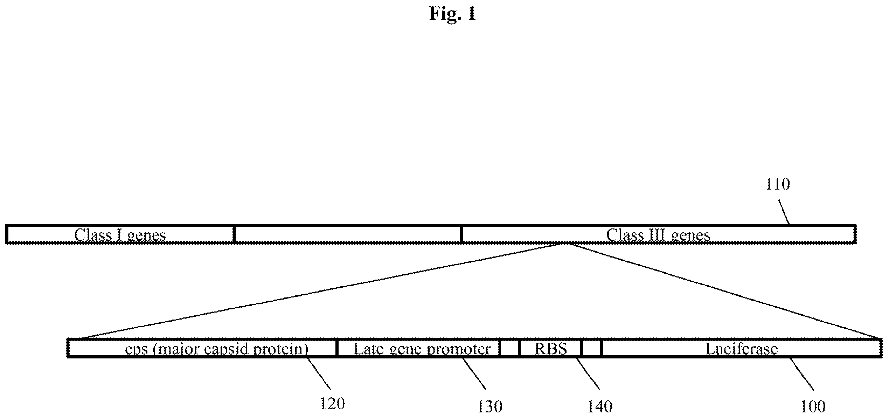

**Figure 1** depicts an indicator phage construct according to an embodiment of the invention illustrating insertion of a genetic construct comprising a luciferase gene, a bacteriophage late gene promoter, and a ribosomal binding site (RBS) inserted into the late (class III) region of a bacteriophage. The promoter depicted is in addition to and separate from the endogenous late gene promoter upstream of the endogenous late genes, such as the gene for major capsid protein (MCP).

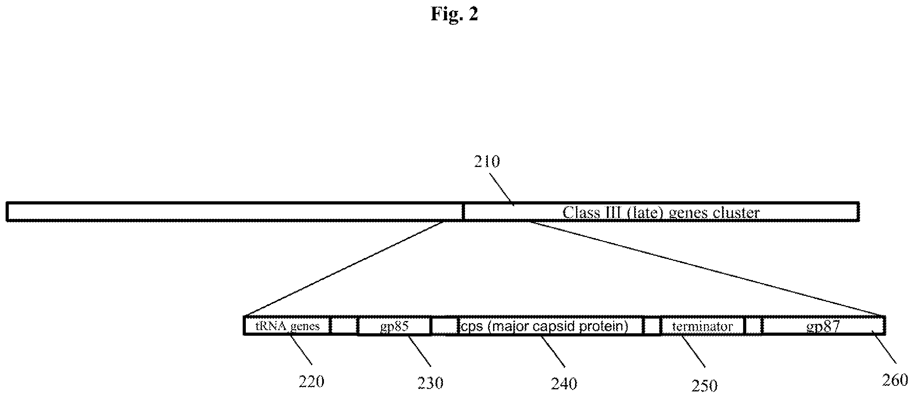

**Figure 2** shows the genome of bacteriophage LMA4, a myovirus (related to Listeria phage LMTA-94) which was obtained from sewage. A hypothetical gene homologous to the putative prohead protease p85protein is upstream of cps, the major capsid gene within the late gene region, consisting of structural genes, which code for virion proteins. Following the cps, is a transcriptional terminator, followed by a homolog to the LMTA-94 tail sheath protein (tsh). As these virion proteins are expressed at a very high level, any genes inserted into this region can be expected to have similar expression levels, as long as late gene promoters and/or other similar control elements are used.

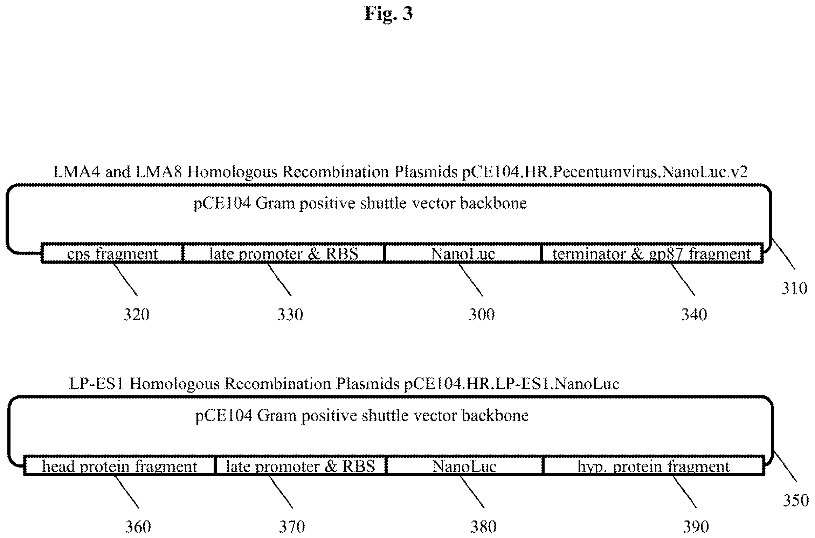

**Figure 3** shows two homologous recombination plasmid construct designs carrying the luciferase gene used to construct the recombinant phages with approximately several hundred basepairs of matching phage sequence upstream and downstream of the insertion site to promote homologous recombination. NANOLUC^{®} luciferase is inserted into a pCE104 Gram positive shuttle vector plasmid backbone with an upstream untranslated region containing a dedicated phage late gene promoter and Ribosomal Entry Site. pCE104.HR.A511.NanoLuc.v2 was used to construct recombinants for the Pecentumviruses A511, LMA4 and LMA8. pCE104.HR.LP-ES1.NanoLuc was used to construct the Homburvirus LP-ES1. Each construct consisted of 500 bp of homologous sequence consisting of a fragment of the Major Capsid Protein gene (cps) followed by a late gene promoter, which was added in addition to the endogenous late gene promoter upstream of the major capsid protein in the phage genome, the luciferase gene, and approximately 258 bp of downstream matching sequence for homologous recombination for pCE104.HR.A511.NanoLuc.v2 and 500 bp of downstream for pCE104.HR.LP-ES1.NanoLuc. All recombinants used a *P100virus* late gene promoter instead of the T4 late gene promoter.

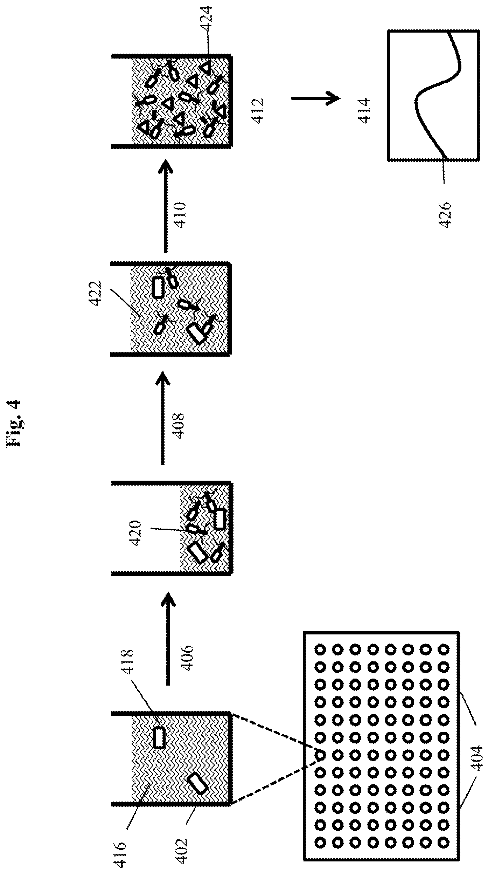

**Figure 4** depicts a filter plate assay for detecting bacteria of interest using a modified bacteriophage according to an embodiment of the invention where bacteria and recombinant phage are incubated on filter plates and after generation of progeny bacteriophage the indicator protein is detected directly without removal of the incubation medium.

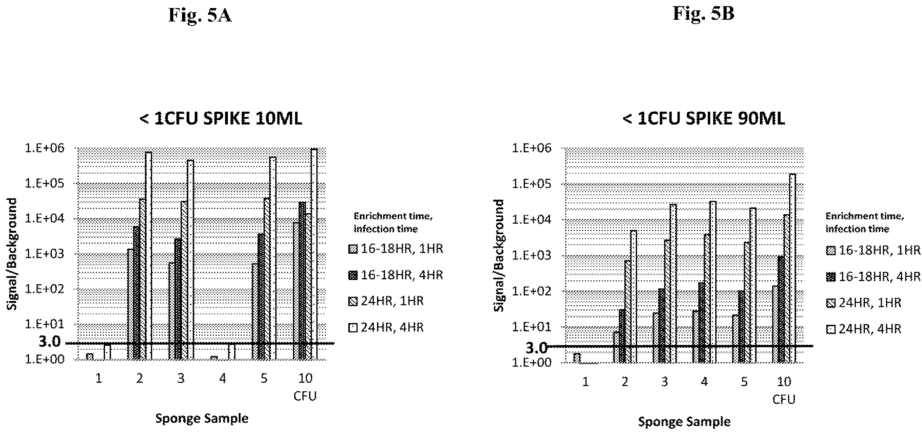

**Figures 5A and 5B** show data from embodiments of a *Listeria* detection assays using recombinant bacteriophage specific for *Listeria* to detect *Listeria* in spiked sponges, using 10 mL added medium (5A) or 90 mL added medium (5B).

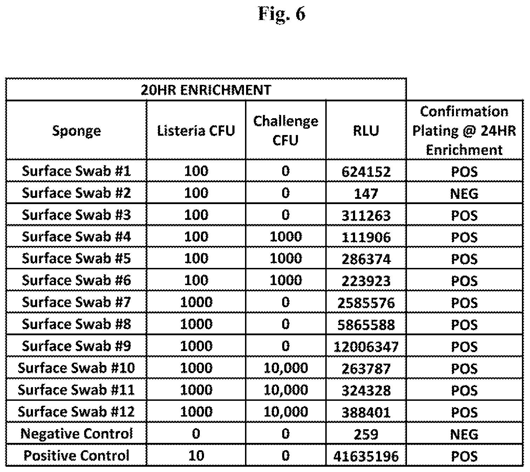

**Figure 6** shows data from embodiments of a *Listeria* detection assay using recombinant bacteriophage specific for *Listeria* to detect *Listeria* in environmental surface swab sponge samples.

### DETAILED DESCRIPTION OF THE INVENTION

Disclosed herein are compositions, methods and systems that demonstrate surprising sensitivity for detection of *Listeria spp.,* in test samples (e.g., biological, food, water, and environmental samples). Detection can be achieved in a shorter timeframe than was previously thought possible using genetically modified infectious agents in assays performed with minimal enrichment times during which microorganisms could potentially multiply. Also surprising is the success of using a potentially high multiplicity of infection (MOI), or high concentrations of plaque forming units (PFU), for incubation with a test sample. Such high phage concentrations (PFU/mL) were previously purported to be detrimental in bacterium detection assays, as they were purported to cause "lysis from without." However, a high concentration of phage can facilitate finding, binding, and infecting a low number of target cells.

The compositions, methods, systems and kits of the invention comprise a cocktail composition comprising at least one recombinant bacteriophage constructed from LMA4, comprising an indicator gene, and an exogenous promoter controlling transcription of the indicator gene, inserted into a late gene region of a bacteriophage genome, wherein the indicator gene is not contiguous with a gene encoding a structural bacteriophage protein and does not yield a fusion protein, and wherein expression of the indicator gene results in an indicator protein product, and wherein the recombinant bacteriophage specifically infects *Listeria spp* for use in detection of *Listeria spp.* Expression of the indicator gene during bacteriophage replication following infection of a host bacterium results in production of a soluble indicator protein product. The recombinant bacteriophage is constructed from LMA4 bacteriophages.

The compositions, methods, systems and kits of the invention comprise the cocktail recited in the claims for use in detection of the microorganisms *Listeria spp.*

In some aspects, the invention comprises a method for detecting a microorganism of interest. The method uses an infectious agent for detection of the microorganism of interest *Listeria spp.* The infections agent is a cocktail composition, the cocktail composition comprising at least one recombinant bacteriophage constructed from LMA4, comprising an indicator gene, and an exogenous promoter controlling transcription of the indicator gene, inserted into a late gene region of a bacteriophage genome, wherein the indicator gene is not contiguous with a gene encoding a structural bacteriophage protein and does not yield a fusion protein, and wherein expression of the indicator gene results in an indicator protein product. The microorganism of interest is *Listeria spp.* and the infectious agent is the bacteriophage that specifically infects a *Listeria spp.* The method comprises detection of a bacterium of interest in a sample by incubating the sample with the cocktail composition that infects the bacterium of interest. The method comprises detecting the indicator protein product, wherein positive detection of the indicator protein product indicates that *Listeria spp.* is present in the sample. In some embodiments, the indicator protein is soluble.

In certain embodiments, the invention may comprise a system. The system is defined as in the apended claims.

Thus, some embodiments of the present invention solve a need by using bacteriophage-based methods for amplifying a detectable signal indicating the presence of the bacteria *Listeria spp..* In certain embodiments as few as 10 bacteria are detected. Because of numerous binding sites for an infectious agent on the surface of *Listeria spp.,* the capacity to produce progeny during infection, and the potential for high level expression of an encoded indicator moiety, the infectious agent or an indicator moiety can be more readily detectable than the *listeria spp.* itself. In this way, embodiments of the present invention can achieve tremendous signal amplification from even a single infected cell.

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. Known methods and techniques are generally performed according to conventional methods well known in the art and as described in various general and more specific references that are discussed throughout the present specification unless otherwise indicated. Enzymatic reactions and purification techniques are performed according to manufacturer's specifications, as commonly accomplished in the art or as described herein. The nomenclatures used in connection with the laboratory procedures and techniques described herein are those well-known and commonly used in the art.

The following terms, unless otherwise indicated, shall be understood to have the following meanings:
As used herein, the terms "a", "an", and "the" can refer to one or more unless specifically noted otherwise.

The use of the term "or" is used to mean "and/or" unless explicitly indicated to refer to alternatives only or the alternatives are mutually exclusive, although the disclosure supports a definition that refers to only alternatives and "and/or." As used herein "another" can mean at least a second or more.

Throughout this application, the term "about" is used to indicate that a value includes the inherent variation of error for the device, the method being employed to determine the value, or the variation that exists among samples.

The term "solid support" or "support" means a structure that provides a substrate and/or surface onto which biomolecules may be bound. For example, a solid support may be an assay well (i.e., such as a microtiter plate or multi-well plate), or the solid support may be a location on a filter, an array, or a mobile support, such as a bead or a membrane (e.g., a filter plate, latex particles, paramagnetic particles, or lateral flow strip).

The term "binding agent" refers to a molecule that can specifically and selectively bind to a second (i.e., different) molecule of interest. The interaction may be non-covalent, for example, as a result of hydrogen bonding, van der Waals interactions, or electrostatic or hydrophobic interactions, or it may be covalent. The term "soluble binding agent" refers to a binding agent that is not associated with (i.e., covalently or non-covalently bound) to a solid support.

As used herein, an "analyte" refers to a molecule, compound or cell that is being measured. The analyte of interest may, in certain embodiments, interact with a binding agent. As described herein, the term "analyte" may refer to a protein or peptide of interest. An analyte may be an agonist, an antagonist, or a modulator. Or, an analyte may not have a biological effect. Analytes may include small molecules, sugars, oligosaccharides, lipids, peptides, peptidomimetics, organic compounds and the like.

The term "detectable moiety" or "detectable biomolecule" or "reporter" or "indicator" or "indicator moiety" refers to a molecule that can be measured in a quantitative assay. For example, an indicator moiety may comprise an enzyme that may be used to convert a substrate to a product that can be measured. An indicator moiety may be an enzyme that catalyzes a reaction that generates bioluminescent emissions (e.g., luciferase). Or, an indicator moiety may be a radioisotope that can be quantified. Or, an indicator moiety may be a fluorophore. Or, other detectable molecules may be used.

As used herein, "bacteriophage" or "phage" includes one or more of a plurality of bacterial viruses. In this disclosure, the terms "bacteriophage" and "phage" include viruses such as mycobacteriophage (such as for TB and paraTB), mycophage (such as for fungi), mycoplasma phage, and any other term that refers to a virus that can invade living bacteria, fungi, mycoplasma, protozoa, yeasts, and other microscopic living organisms and uses them to replicate itself. Here, "microscopic" means that the largest dimension is one millimeter or less. Bacteriophages are viruses that have evolved in nature to use bacteria as a means of replicating themselves. A phage does this by attaching itself to a bacterium and injecting its DNA (or RNA) into that bacterium, and inducing it to replicate the phage hundreds or even thousands of times. This is referred to as phage amplification.

As used herein, "late gene region" refers to a region of a viral genome that is transcribed late in the viral life cycle. The late gene region typically includes the most abundantly expressed genes (e.g., structural proteins assembled into the bacteriophage particle). Late genes are synonymous with class III genes and include genes with structure and assembly functions. For example, the late genes (synonymous with class III,) are transcribed in phage T7, e.g., from 8 minutes after infection until lysis, class I (e.g., RNA polymerase) is early from 4-8 minutes, and class II from 6-15 minutes, so there is overlap in timing of II and III. A late promoter is one that is naturally located and active in such a late gene region.

As used herein, "culturing for enrichment" refers to traditional culturing, such as incubation in media favorable to propagation of microorganisms, and should not be confused with other possible uses of the word "enrichment," such as enrichment by removing the liquid component of a sample to concentrate the microorganism contained therein, or other forms of enrichment that do not include traditional facilitation of microorganism propagation. Culturing for enrichment for periods of time may be employed in some embodiments of methods described herein.

As used herein "recombinant" refers to genetic (i.e., nucleic acid) modifications as usually performed in a laboratory to bring together genetic material that would not otherwise be found. This term is used interchangeably with the term "modified" herein.

As used herein "RLU" refers to relative light units as measured by a luminometer (e.g., GLOMAX^{®} 96) or similar instrument that detects light. For example, the detection of the reaction between luciferase and appropriate substrate (e.g., NANOLUC^{®} with NANO-GLO^{®}) is often reported in RLU detected.

As used herein "time to results" refers to the total amount of time from beginning of sample incubation to generated result. Time to results does not include any confirmatory testing time. Data collection can be done at any time after a result has been generated.

### Samples

Each of the embodiments of the methods and systems of the invention can allow for the rapid detection and quantification of *Listeria spp.* in a sample according to the appended claims.

Samples may be liquid, solid, or semi-solid. Samples may be swabs of solid surfaces. Samples may include environmental materials, such as water samples, or the filters from air samples or aerosol samples from cyclone collectors. Samples may be of vegetables, meat, fish, poultry, peanut butter, processed foods, powdered infant formula, powdered milk, teas, starches, eggs, milk, cheese, or other dairy products.

In some embodiments, samples may be used directly in the detection methods of the present invention, without preparation, concentration, or dilution. For example, liquid samples, including but not limited to, milk and juices, may be assayed directly. Samples may be diluted or suspended in solution, which may include, but is not limited to, a buffered solution or a bacterial culture medium. A sample that is a solid or semi-solid may be suspending in a liquid by mincing, mixing or macerating the solid in the liquid. A sample should be maintained within a pH range that promotes bacteriophage attachment to the host bacterial cell. A sample should also contain the appropriate concentrations of divalent and monovalent cations, including but not limited to Na⁺, Mg²⁺, and Ca²⁺. Preferably a sample is maintained at a temperature that maintains the viability of any pathogen cells contained within the sample.

In some embodiments of the detection assay, the sample is maintained at a temperature that maintains the viability of any pathogen cell present in the sample. For example, during steps in which bacteriophages are attaching to bacterial cells, it is preferable to maintain the sample at a temperature that facilitates bacteriophage attachment. During steps in which bacteriophages are replicating within an infected bacterial cell or lysing such an infected cell, it is preferable to maintain the sample at a temperature that promotes bacteriophage replication and lysis of the host. Such temperatures are at least about 25 degrees Celsius (C), more preferably no greater than about 35 degrees C, most preferably about 30 degrees C.

Assays may include various appropriate control samples. For example, control samples containing no bacteriophages or control samples containing bacteriophages without bacteria may be assayed as controls for background signal levels.

### Indicator Bacteriophage

As described in more detail herein, the compositions, methods, systems and kits of the invention comprise a cocktail composition comprising at least one recombinant bacteriophage constructed from LMA4, comprising an indicator gene, and an exogenous promoter controlling transcription of the indicator gene, inserted into a late gene region of a bacteriophage genome, wherein the indicator gene is not contiguous with a gene encoding a structural bacteriophage protein and does not yield a fusion protein, and wherein expression of the indicator gene results in an indicator protein product, and wherein the recombinant bacteriophage specifically infects *Listeria spp...*

A recombinant indicator bacteriophage can include a reporter or indicator gene. In embodiments of the invention, the indicator gene does not encode a fusion protein. Expression of the indicator gene during bacteriophage replication following infection of a host bacterium results in a soluble indicator protein product. In embodiments, the indicator gene is inserted into a late gene region of the bacteriophage. Late genes are generally expressed at higher levels than other phage genes, as they code for structural proteins. The late gene region may be a class III gene region and may include a gene for a major capsid protein.

Some embodiments that do not form part of the claimed invention include designing (and optionally preparing) a sequence for homologous recombination downstream of the major capsid protein gene. Other embodiments that do not form part of the claimed invention include designing (and optionally preparing) a sequence for homologous recombination upstream of the major capsid protein gene. In some embodiments, the sequence comprises a codon-optimized reporter gene preceded by an untranslated region. The untranslated region may include a phage late gene promoter and ribosomal entry site.

In embodiments, an indicator bacteriophage is derived from *Listeria-specific* phage. In some embodiments, the selected wild-type bacteriophage or cocktail of wild-type bacteriophages is capable of infecting at least one target *Listeria spp. Listeria* species are ubiquitous in the environment and are often found in water, sewage and soil. In some embodiments, the selected wild-type bacteriophage or cocktail of wild-type bacteriophages is capable of infecting one or more, two or more, or three or more target *Listeria spp.* In certain instances the target species of *Listeria* is selected from *L. monocytogenes*, *L. ivanovii*, and *L. grayi.*

In embodiments of the invention, the cocktail composition comprises at least one recombinant bacteriophage constructed from LMA4. In some embodiments, further selected wild-type bacteriophage is from the *Caudovirales* order of phages. *Caudovirales* are an order of tailed bacteriophages with double-stranded DNA (dsDNA) genomes. Each virion of the *Caudovirales* order has an icosahedral head that contains the viral genome and a flexible tail. The *Caudovirales* order comprises five bacteriophage families: *Myoviridae* (long contractile tails), *Siphoviridae* (long non-contractile tails), *Podoviridae* (short non-contractile tails), *Ackermannviridae*, and *Herelleviridae.* The term myovirus can be used to describe any bacteriophage with an icosahedral head and a long contractile tail, which encompasses bacteriophages within both the *Myoviridae* and *Herelleviridae* families. In some embodiments, further selected wild-type bacteriophage is a member of the *Myoviridae* family such as, *Listeria* phage B054, *Listeria* phage LipZ5, *Listeria* phage PSU-VKH-LP041, and *Listeria* phage WIL-2. In other embodiments, further selected wild-type bacteriophage is a member of the family *Herelleviridae.* The genus *Pecentumvirus,* under the family *Herelleviridae* includes bacteriophages such as *Listeria* phage LMSP-25, *Listeria* phage LMTA-148, *Listeria* phage LMTA-34, *Listeria* phage LP-048, *Listeria* phage LP-064, *Listeria* phage LP-083-2, *Listeria* phage LP-125, Listeria virus P100, *Listeria* phage List-36, *Listeria* phage WIL-1, *Listeria* phage vB_LmoM_AG20, and *Listeria* virus A511. LMA4 and LMA8 are also likely in the genus *pecentumvirus*, under the family *Herelleviridae.* In other embodiments, further selected wild-type bacteriophage is LMA8. In certain instances further selected wild-type bacteriophage is LP-ES3A, which is derived from A511 but has been adapted to be capable of infecting serotype 3A of *Listeria monocytogenes.* In still other embodiments, further selected wild-type bacteriophage is a member of the family *Ackermannviridae.* In still other embodiments, further selected wild-type bacteriophage is a member of the family *Siphoviridae*, which includes *Listeria* phages A006, A118, A500, B025, LP-026, LP-030-2, LP-030-3, LP-037, LP-101, LP-110, LP-114, P35, P40, P70, PSA, vB_LmoS_188, and vB_Lmos_293. In other embodiments, further selected wild-type bacteriophage is LP-ES1. LP-ES1 is also likely in the genus *Homburgvirus*, under the family *Siphoviridae.*

In embodiments, indicator bacteriophage is derived from *Listeria-specific* phage; the cocktail composition comprises at least one recombinant bacteriophage constructed from LMA4. An indicator bacteriophage may be constructed from a Pecentumvirus, Tequatravirus, ViI, Kuttervirus, Homburgvirus, LMTA-94, LMA4, LMA8, P70, LP-ES1, LP-ES3A or another bacteriophage having a genome with at least 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 % homology to *Listeria* phage LMTA-94, P70, T7, T7-like, T4, T4-like, *Listeria* spp.-specific bacteriophage, ViI, or ViI-like (Kuttervirus, per GenBank/NCBI) bacteriophages. In other embodiments, further selected wild-type bacteriophage is LP-ES1, LP-ES3A, or LMA8. In embodiments, the indicator phage is derived from a bacteriophage that is highly specific for a particular pathogenic microorganism. The genetic modifications may avoid deletions of wild-type genes and thus the modified phage may remain more similar to the wild-type infectious agent than many commercially available phage. Environmentally derived bacteriophage may be more specific for bacteria that are found in the environment and as such, genetically distinct from phage available commercially.

In another aspect of the invention, a cocktail composition comprises at least one type of recombinant bacteriophage constructed from LMA4. In some embodiments, the cocktail composition comprises further types of recombinant bacteriophage constructed from LMA8, A511, P70, LP-ES1, and LP-ES3A. In other embodiments, the cocktail composition comprises further types of recombinant bacteriophage constructed from LMA8, LP-ES1, and LP-ES3A.

Moreover, phage genes thought to be nonessential may have unrecognized function. For example, an apparently nonessential gene may have an important function in elevating burst size such as subtle cutting, fitting, or trimming functions in assembly. Therefore, deleting genes to insert an indicator may be detrimental. Most phages can package DNA that is a few percent larger than their natural genome. With this consideration, a smaller indicator gene may be a more appropriate choice for modifying a bacteriophage, especially one with a smaller genome. OpLuc and NANOLUC^{®} proteins are only about 20 kDa (approximately 500-600 bp to encode), while FLuc is about 62 kDa (approximately 1,700 bp to encode). Moreover, the reporter gene should not be expressed endogenously by the bacteria (i.e., is not part of the bacterial genome), should generate a high signal to background ratio, and should be readily detectable in a timely manner. In some embodiments, the indicator gene is a luciferase. In other embodiments, the indicator gene is an active subunit of a luciferase. Promega's NANOLUC^{®} is a modified *Oplophorus gracilirostris* (deep sea shrimp) luciferase. In some embodiments, NANOLUC^{®} combined with Promega's NANO-GLO^{®}, an imidazopyrazinone substrate (furimazine), can provide a robust signal with low background.

In some indicator phage embodiments, the indicator gene can be inserted into an untranslated region to avoid disruption of functional genes, leaving wild-type phage genes intact, which may lead to greater fitness when infecting non-laboratory strains of bacteria. Additionally, including stop codons in all three reading frames may help to increase expression by reducing read-through, also known as leaky expression. This strategy may also eliminate the possibility of a fusion protein being made at low levels, which would manifest as background signal (e.g., luciferase) that cannot be separated from the phage.

An indicator gene may express a variety of biomolecules. The indicator gene is a gene that expresses a detectable product or an enzyme that produces a detectable product. For example, in one embodiment the indicator gene encodes a luciferase enzyme. Various types of luciferase may be used. In alternate embodiments, and as described in more detail herein, the luciferase is one of *Oplophorus* luciferase, Firefly luciferase, Lucia luciferase, Renilla luciferase, or an engineered luciferase. In some embodiments, the luciferase gene is derived from *Oplophorus.* In some embodiments, the indicator gene is a genetically modified luciferase gene, such as NANOLUC^{®}.

Thus, in some embodiments, the present invention comprises a genetically modified bacteriophage comprising a non-bacteriophage indicator gene in the late (class III) gene region. In some embodiments, the non-native indicator gene is under the control of a late promoter. Using a viral late gene promoter ensures the reporter gene (e.g., luciferase) is not only expressed at high levels, like viral capsid proteins, but also does not shut down like endogenous bacterial genes or even early viral genes.

In some embodiments, the late promoter is a Pecentumvirus, Tequatravirus, Homburgvirus, or Kuttervirus promoter, or another phage promoter similar to that found in the selected wild-type phage, i.e., without genetic modification. The late gene region may be a class III gene region, and the bacteriophage may be derived from *Listeria* phage LMTA-94, P70, A511, LP-ES1, LP-ES3A, LMA4, LMA8, Pecentumvirus, Tequatravirus, Homburgvirus, Kuttervirus, T7, T4, T4-like, ViI, *Listeria* spp.-specific bacteriophage, or another wild-type bacteriophage having a genome with at least 70, 75, 80, 85, 90 or 95% homology to LMTA-94, LMA4, LMA8, Pecentumvirus, Tequatravirus, Homburgvirus, Kuttervirus, T7, T4, ViI, or *Listeria-specific* bacteriophage. The *Pecentumvirus* late gene promoter is distinct from the T4 or Tequatravirus promoter, as it consists of not only the -10 region, but also a -35 region. This -35 region differs from the standard -35 region found in most bacterial promoters.

Genetic modifications to infectious agents may include insertions, deletions, or substitutions of a small fragment of nucleic acid, a substantial part of a gene, or an entire gene. In some embodiments, inserted or substituted nucleic acids comprise non-native sequences. A non-native indicator gene may be inserted into a bacteriophage genome such that it is under the control of a bacteriophage promoter. Thus, in embodiments, the non-native indicator gene is not part of a fusion protein. That is, in some embodiments, a genetic modification may be configured such that the indicator protein product does not comprise polypeptides of the wild-type bacteriophage. In some embodiments, the indicator protein product is soluble. In embodiments, the invention comprises a method for detecting a bacterium of interest comprising the step of incubating a test sample with such a recombinant bacteriophage.

In embodiments, expression of the indicator gene in progeny bacteriophage following infection of host bacteria results in a free, soluble protein product. In embodiments, the non-native indicator gene is not contiguous with a gene encoding a structural phage protein and therefore does not yield a fusion protein. Unlike systems that employ a fusion of a detection moiety to the capsid protein (i.e., a fusion protein), embodiments of the present invention express a soluble indicator or reporter (e.g., soluble luciferase). In some embodiments, the indicator or reporter is ideally free of the bacteriophage structure. That is, the indicator or reporter is not attached to the phage structure. As such, the gene for the indicator or reporter is not fused with other genes in the recombinant phage genome. This may greatly increase the sensitivity of the assay (down to a single bacterium), and simplify the assay, allowing the assay to be completed in two hours or less for some embodiments, as opposed to several hours due to additional purification steps required with constructs that produce detectable fusion proteins. Further, fusion proteins may be less active than soluble proteins due, e.g., to protein folding constraints that may alter the conformation of the enzyme active site or access to the substrate. If the concentration is 1,000 bacterial cells/mL of sample, for example, less than four hours of infection may be sufficient for the detection of the target bacterium.

Moreover, fusion proteins by definition limit the number of the moieties attached to subunits of a protein in the bacteriophage. For example, using a commercially available system designed to serve as a platform for a fusion protein would result in about 415 copies of the fusion moiety, corresponding to the about 415 copies of the gene 10B capsid protein in each T7 bacteriophage particle. Without this constraint, infected bacteria can be expected to express many more copies of the detection moiety (e.g., luciferase) than can fit on the bacteriophage. Additionally, large fusion proteins, such as a capsid-luciferase fusions, may inhibit assembly of the bacteriophage particle, thus yielding fewer bacteriophage progeny. Thus a soluble, non-fusion indicator gene product is preferable.

In some embodiments, the indicator phage encodes a reporter, such as a detectable enzyme. The indicator gene product may generate light and/or may be detectable by a color change. Various appropriate enzymes are commercially available, such as alkaline phosphatase (AP), horseradish peroxidase (HRP), or luciferase (Luc). In some embodiments, these enzymes may serve as the indicator moiety. In some embodiments, Firefly luciferase is the indicator moiety. In some embodiments, *Oplophorus* luciferase is the indicator moiety. In some embodiments, NANOLUC^{®} is the indicator moiety. Other engineered luciferases or other enzymes that generate detectable signals may also be appropriate indicator moieties.

In embodiments, the use of a soluble detection moiety eliminates the need to remove contaminating parental phage from the lysate of the infected sample cells. With a fusion protein system, any bacteriophage used to infect sample cells would have the detection moiety attached, and would be indistinguishable from the daughter bacteriophage also containing the detection moiety. As detection of sample bacteria relies on the detection of a newly created (*de novo* synthesized) detection moiety, using fusion constructs requires additional steps to separate old (parental) moieties from newly created (daughter bacteriophage) moieties. This may be accomplished by washing the infected cells multiple times, prior to the completion of the bacteriophage life cycle, inactivating excess parental phage after infection by physical or chemical means, and/or chemically modifying the parental bacteriophage with a binding moiety (such as biotin), which can then be bound and separated (such as by Streptavidin-coated Sepharose beads). However, even with all these attempts at removal, parental phage can remain when a high concentration of parental phage is used to assure infection of a low number of sample cells, creating background signal that may obscure detection of signal from infected cell progeny phage.

By contrast, with the soluble detection moiety expressed in embodiments of the present invention, purification of the parental phage from the final lysate is unnecessary, as the parental phage do not have any detection moiety attached. Thus any detection moiety present after infection must have been created *de novo*, indicating the presence of an infected bacterium or bacteria. To take advantage of this benefit, the production and preparation of parental phage may include purification of the phage from any free detection moiety produced during the production of parental bacteriophage in bacterial culture. Standard bacteriophage purification techniques may be employed to purify some embodiments of phage according to the present invention, such as sucrose density gradient centrifugation, cesium chloride isopycnic density gradient centrifugation, HPLC, size exclusion chromatography, and dialysis or derived technologies (such as Amicon brand concentrators - Millipore, Inc.). Cesium chloride isopycnic ultracentrifugation can be employed as part of the preparation of recombinant phage of the invention, to separate parental phage particles from contaminating luciferase protein produced upon propagation of the phage in the bacterial host. In this way, the parental recombinant bacteriophage of the invention is substantially free of any luciferase generated during production in the bacteria. Removal of residual luciferase present in the phage stock can substantially reduce background signal observed when the recombinant bacteriophage are incubated with a test sample.

In some embodiments of modified bacteriophage, the late promoter (class III promoter, e.g., from Pecentumvirus, Homburgvirus, T7, T4, ViI, or LMA4/8) has high affinity for RNA polymerase of the same bacteriophage that transcribes genes for structural proteins assembled into the bacteriophage particle. These proteins are the most abundant proteins made by the phage, as each bacteriophage particle comprises dozens or hundreds of copies of these molecules. The use of a viral late promoter can ensure optimally high level of expression of the luciferase detection moiety. The use of a late viral promoter derived from, specific to, or active under the original wild-type bacteriophage the indicator phage is derived from (e.g., a Pecentumvirus, Homburgvirus, T4, T7, ViI, or LMA4/8 late promoter with a Pecentumvirus, T4, T7-, ViI-, or LMA-based system) can further ensure optimal expression of the detection moiety. The use of a standard bacterial (non-viral/non-bacteriophage) promoter may in some cases be detrimental to expression, as these promoters are often down-regulated during bacteriophage infection (in order for the bacteriophage to prioritize the bacterial resources for phage protein production). Thus, in embodiments, the phage is preferably engineered to encode and express at high level a soluble (free) indicator moiety, using a placement in the genome that does not limit expression to the number of subunits of a phage structural component.

Compositions of the invention may comprise one or more wild-type or genetically modified infectious agents (e.g., bacteriophages) and one or more indicator genes. In some embodiments, compositions can include cocktails of different indicator phages that may encode and express the same or different indicator proteins. In some embodiments, the cocktail of bacteriophage comprises at least two different types of recombinant bacteriophages.

### Methods of Preparing Indicator Bacteriophage

Embodiments of methods for making indicator bacteriophage begin with selection of a wild-type bacteriophage for genetic modification. Some bacteriophage are highly specific for a target bacterium. This presents an opportunity for highly specific detection.

Thus, the methods of the present invention utilize the high specificity of binding agents, associated with infectious agents that recognize and bind to a particular microorganism of interest as a means to amplify a signal and thereby detect low levels of a microorganism (e.g., a single microorganism) present in a sample. For example, infectious agents (bacteriophage) specifically recognize surface receptors of particular microorganisms and thus specifically infect those microorganisms. As such, these infectious agents are appropriate binding agents for targeting a microorganism of interest.

Embodiments of the invention utilize the specificity of binding and high-level genetic expression capacity of recombinant bacteriophage for rapid and sensitive targeting to infect and facilitate detection of a bacterium of interest. In embodiments, a *Listeria-specific* bacteriophage LMA4 is genetically modified to include a reporter gene. In embodiments the late gene region of a bacteriophage is genetically modified to include a reporter gene. In some embodiments, a reporter gene is positioned downstream of the major capsid gene. In other embodiments, a reporter gene is positioned upstream of the major capsid gene. In embodiments, the inserted genetic construct further comprises its own exogenous, dedicated promoter to drive expression of the indicator gene. The exogenous promoter is in addition to any endogenous promoter in the phage genome. As bacteriophage produce polycistronic mRNA transcripts, only a single promoter is required upstream of the first gene/cistron in the transcript. Conventional recombinant constructs only use the endogenous bacteriophage promoter to drive inserted genes. In contrast, addition of an additional promoter upstream of the reporter gene and ribosomal binding site may increase gene expression by acting as a secondary initiation site for transcription. The complicated and compact genomes of viruses often have overlapping genes in different frames, sometimes in two different directions.

Some embodiments of methods, that do not form part of the claimed invention, for preparing a recombinant indicator bacteriophage include selecting a wild-type bacteriophage that specifically infects a target pathogenic bacterium such as *Listeria spp*.; preparing a homologous recombination plasmid/vector that comprises an indicator gene; transforming the homologous recombination plasmid/vector into target pathogenic bacteria; infecting the transformed target pathogenic bacteria with the selected wild-type bacteriophage, thereby allowing homologous recombination to occur between the plasmid/vector and the bacteriophage genome; and isolating a particular clone of recombinant bacteriophage.

Various methods for designing and preparing a homologous recombination plasmid are known. Various methods for transforming bacteria with a plasmid are known, including heat-shock, F pilus-mediated bacterial conjugation, electroporation, and other methods. Various methods for isolating a particular clone following homologous recombination are also known. Some method embodiments described herein utilize particular strategies.

Thus, some embodiments of methods, that do not form part of the claimed invention, for preparing indicator bacteriophage include the steps of selecting a wild-type bacteriophage that specifically infects a target pathogenic bacterium; determining the natural sequence in the late region of the genome of the selected bacteriophage; annotating the genome and identifying the major capsid protein gene of the selected bacteriophage; designing a sequence for homologous recombination adjacent to the major capsid protein gene, wherein the sequence comprises a codon-optimized reporter gene; incorporating the sequence designed for homologous recombination into a plasmid/vector; transforming the plasmid/vector into target pathogenic bacteria; selecting for the transformed bacteria; infecting the transformed bacteria with the selected wild-type bacteriophage, thereby allowing homologous recombination to occur between the plasmid and the bacteriophage genome; determining the titer of the resulting recombinant bacteriophage lysate; and performing a limiting dilution assay to enrich and isolate the recombinant bacteriophage. Some embodiments comprise further repeating the limiting dilution and titer steps, following the first limiting dilution assay, as needed until the recombinant bacteriophage represent a detectable fraction of the mixture. For example, in some embodiments the limiting dilution and titer steps can be repeated until at least 1/30 of the bacteriophage in the mixture are recombinant before isolating a particular clone of recombinant bacteriophage. A ratio of 1:30 recombinant: wild-type is expected, in some embodiments, to yield an average of 3.2 transducing units (TU) per 96 plaques (e.g., in a 96-well plate). The initial ratio of recombinant to wild-type phage may be determined by performing limiting dilution assays based on the TCID50 (tissue culture infectious dose 50%) as previously described in U.S. App. No. 15/409,258. By Poisson distribution, a 1:30 ratio generates a 96% chance of observing at least one TU somewhere in the 96 wells.

**Figure 1** depicts a schematic representation of the genomic structure of a recombinant indicator bacteriophage of the invention. For the embodiment depicted in **Figure 1****,** the detection moiety is encoded by a luciferase gene **100** inserted within the late (class III) gene region **110,** which is expressed late in the viral life cycle. Late genes are generally expressed at higher levels than other phage genes, as they code for structural proteins. Thus, in the embodiment of the recombinant phage depicted in **Figure 1****,** the indicator gene (i.e., luciferase) is inserted into the late gene region, just after the gene for major capsid protein (cps) **120,** and is a construct comprising the luciferase gene **100.** In some embodiments, the construct depicted in **Figure 1** may include stop codons in all 3 reading frames to ensure luciferase is not incorporated into the cps gene product through creation of a fusion protein. Also as depicted in **Figure 1****,** the construct may comprise an additional, dedicated late promoter **130** to drive transcription and expression of the luciferase gene. The construct also comprises a ribosome binding site (RBS) **140.** This construct ensures soluble luciferase is produced such that expression is not limited to the number of capsid proteins inherent in the phage display system.

As noted herein, in certain embodiments, it may be preferred to utilize infectious agents that have been isolated from the environment for production of the infectious agents of the invention. In this way, infectious agents that are specific to naturally derived microorganisms may be generated.

For example, **Figure 2** shows the genome of bacteriophage LMA4, a wild-type bacteriophage that specifically infects *Listeria spp.* As discussed in the Examples, the Major Capsid Protein (cps) **240** and various other structural genes are within the late gene region **210,** consisting of structural genes, which code for virion proteins. Genes coding for tRNA **220** represent genomic sequence adjacent to, but outside of the late gene region. A hypothetical gene homologous to the putative prohead protease of *Listeria* phage LMTA-94 **230** is upstream of cps **240,** consisting of structural genes, which code for virion proteins. Other late genes depicted are homologs to *Listeria* phage LMTA-94's putative Major Capsid Protein (cps) **240,** followed by a transcriptional terminator 250, and a homolog to *Listeria* phage LMTA-94 Tail Sheath Protein (tsh) **260.** As these virion proteins are expressed at a very high level, any genes inserted into this region can be expected to have similar expression levels, as long as late gene promoters and/or other similar control elements are used.

There are numerous known methods and commercial products for preparing plasmids. For example, PCR, site-directed mutagenesis, restriction digestion, ligation, cloning, and other techniques may be used in combination to prepare plasmids. Synthetic plasmids can also be ordered commercially (e.g., GeneWiz). Cosmids can also be employed, or the CRISPR/CAS9 system could be used to selectively edit a bacteriophage genome. Some embodiments of methods of preparing a recombinant indicator bacteriophage include designing a plasmid that can readily recombine with the wild-type bacteriophage genome to generate recombinant genomes. In designing a plasmid, some embodiments include addition of a codon-optimized reporter gene, such as a luciferase gene. Some embodiments further include addition of elements into the upstream untranslated region. For example, in designing a plasmid to recombine with the *Listeria* -specific bacteriophage genome, an upstream untranslated region can be added between the sequence encoding the C-terminus of the gp23 / Major Capsid Protein and the start codon of the NANOLUC^{®} reporter gene. The untranslated region can include a promoter, such as a T4, Tequatravirus, Homburgvirus, T7, T7-like, Pecentumvirus, *Listeria-specific* bacteriophage, ViI, or Kuttervirus promoter. The untranslated region can also include a Ribosomal Entry / Binding Site (RBS), also known as a "Shine-Dalgarno Sequence" with bacterial systems. Either or both of these elements, or other untranslated elements, can be embedded within a short upstream untranslated region made of random sequences comprising about the same GC content as rest of the phage genome. The random region should not include an ATG sequence, as that will act as a start codon.

The cocktail compositions of the invention comprise at least one recombinant bacteriophage constructed from LMA4. The compositions may further comprise various infectious agents and/or indicator genes. For example, **Figure 3** shows a homologous recombination plasmid construct used in making the indicator phage specific for *Listeria spp.* Constructs were made and used in recombination with *Listeria spp.* phage LMA4, *Listeria spp.* phage LMA8, *Listeria spp.* phage LP-ES3A, *Listeria spp.* phage LP-ES1 or other *Listeria-*specific phages to generate recombinant bacteriophage of the invention. The construct in **Figure 3** shows a general schematic for the recombination plasmid used for homologous recombination insertion of the NANOLUC^{®} luciferase into both *Listeria spp. Pecentumvirus* phages LMA4 and LMA8, each with 500 bp of upstream and downstream homologous sequence: homologous recombination plasmid pCE104.HR.ListeriaPhage.NANOLUC.v2. *Pecentumvirus.NANOLUC.v2* and the recombination plasmid used for homologous recombination insert of the NANOLUC^{®} luciferase into *Listeria spp. Homburgvirus* phage LP-ES1, pCE104.HR.LP-ES1.NanoLuc

In certain embodiments a plasmid is designated
*pCE104.HR.Pecentumvirus.NanoLuc.v2.* The detection/indicator moiety is encoded by the NANOLUC^{®} reporter gene **300.** The insert, represented by the series of rectangles, is in the Gram positive shuttle vector, pCE104 **310.** The upstream homologous recombination region consists of 500bp of the major capsid protein C-terminal fragment **320.** A *Pecentumvirus* late promoter consensus sequence & Shine-Dalgarno Ribosomal Entry/Binding Site within the 5' untranslated region **330.** The codon-optimized NANOLUC^{®} reporter gene **300** follows immediately after. The endogenous transcriptional terminator comes next, along with the untranslated region (UTR) and hypothetical protein N-Terminal fragment consisting of the downstream homologous recombination **340** are at the end of the Homologous Recombination region.

The Major Capsid Protein fragment is a part of a structural gene that encodes a virion protein. As these virion proteins are expressed at a very high level, any genes inserted into this region can be expected to have similar expression levels, as long as late gene promoters and/or other similar control elements are used.

In some embodiments, indicator phage according to the invention comprise *Listeria-*specific bacteriophage LMA4 genetically engineered to comprise a reporter gene such as a luciferase gene. For example, an indicator phage can be *Listeria spp.*-specific bacteriophage wherein the genome comprises the sequence of the NANOLUC^{®} gene. A recombinant *Listeria-*specific NanoLuc bacteriophage genome may further comprise a consensus promoter of *Pecentumvirus,* T4, T7, *Listeria-specific,* ViI, LMA4, or LMA8 bacteriophage or another late promoter. In embodiments, the promoter is an exogenous promoter. Insertion of an exogenous promoter to drive expression of an indicator gene is advantageous in that expression is not limited by the expression of other phage proteins (e.g., the major capsid protein).

Thus, in the embodiment of the recombinant phage generated as a result of the recombination, the indicator gene (i.e., NANOLUC^{®}) is inserted into the late gene region, just downstream of the gene encoding the major capsid protein, and thus creates recombinant bacteriophage genomes comprising the NANOLUC^{®} gene. The construct may additionally comprise the consensus promoter of *Listeria* phage LMTA-94, T4, T7, *Listeria-specific* bacteriophage, ViI, or another late promoter or another suitable promoter to drive transcription and expression of the luciferase gene. The construct may also comprise a composite untranslated region synthesized from several UTRs. This construct ensures soluble luciferase is produced such that expression is not limited to the number of capsid proteins inherent in the phage display system.

Recombinant phage generated by homologous recombination of a plasmid designed for recombination with the wild-type phage genome can be isolated from a mixture comprising a very small percentage (e.g., 0.005%) of total phage genomes. Following isolation, large scale production may be performed to obtain high titer recombinant indicator phage stocks appropriate for use in the *Listeria spp.* detection assay. Furthermore, cesium chloride isopycnic density gradient centrifugation may be used to separate phage particles from contaminating luciferase protein to reduce background.

### Methods of Using Infectious Agents for Detecting Listeria spp.

As noted herein, in certain embodiments, the invention may comprise methods of using infectious particles for detecting microorganisms. The methods of the invention may be embodied in a variety of ways.

The invention comprises a method for detecting *Listeria spp.* in a sample comprising: incubating the sample with a cocktail composition comprising at least one *Listeria-specific* recombinant bacteriophage constructed from LMA4; and detecting an indicator protein product produced by the recombinant bacteriophage, wherein positive detection of the indicator protein product indicates that *Listeria spp.* is present in the sample.

In embodiments, at least one type of recombinant bacteriophage is constructed from LMA4. In some embodiments, further recombinant bacteriophages can be constructed from LMA8, A511, P70, LP-ES1, and LP-ES3A. In other embodiments, at least one further type of recombinant bacteriophage is constructed from LMA8, LP-ES1, and LP-ES3A.

In certain embodiments, the assay may be performed to utilize a general concept that can be modified to accommodate different sample types or sizes and assay formats. Embodiments employing recombinant bacteriophage of the invention (i.e., indicator bacteriophage) may allow rapid detection of specific bacterial strains *Listeria* spp., with total assay times under 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12, 12.5, 13.0, 13.5, 14.0, 14.5, 15.0, 15.5, 16.0, 16.5, 17.0, 17.5, 18.0, 18.5, 19.0, 19.5, 20.0, 21.0, 21.5 22.0, 22.5, 23.0, 23.5, 24.0, 24.5 25.0, 25.5, or 26.0 hours, depending on the sample type, sample size, and assay format. For example, the amount of time required may be somewhat shorter or longer depending on the strain of bacteriophage and the strain of bacteria to be detected in the assay, type and size of the sample to be tested, conditions required for viability of the target, complexity of the physical/chemical environment, and the concentration of "endogenous" non-target bacterial contaminants.

The bacteriophage (e.g., T7, T4,P70 P100, A511, LP-ES3A, LP-ES1, LMA4 or LMA8 phage) may be engineered to express a soluble luciferase during replication of the phage. Expression of luciferase is driven by a viral capsid promoter (e.g., the bacteriophage Pecentumvirus or T4 late promoter), yielding high expression. Parental phage are prepared such that they are free of luciferase, so the luciferase detected in the assay must come from replication of progeny phage during infection of the bacterial cells. Thus, there is generally no need to separate out the parental phage from the progeny phage.

**Figure 4** depicts a filter plate assay for detecting *Listeria* using a modified bacteriophage according to an embodiment of the invention. Briefly, samples **416** that include a bacterium of interest **418** may be added to wells **402** of a multi-well filter plate **404** and spun **406** to concentrate the samples by removal of liquid from the sample. Genetically modified phage **420** are added to wells and incubated with additional media added for enough time sufficient for adsorption **408** followed by infection of target bacteria and advancement of the phage life cycle **410** (e.g., ~ 240 minutes). Finally, luciferase substrate is added and reacts with any luciferase present **424.** The resulting emission is measured in a luminometer **414** which detects luciferase activity **426.**

In some embodiments, enrichment of bacteria in the sample is not needed prior to testing. In some embodiments, the sample may be enriched prior to testing by incubation in conditions that encourage growth. In such embodiments, the enrichment period can be 1, 2, 3, 4, 5, 6, 7, 8, 9, 10, 11, 12, 13, 14, 15, 16, 17, 18, 19, 20, 21, 22, 23, or 24 hours or longer, depending on the sample type and size.

In some embodiments, the indicator bacteriophage comprises a detectable indicator moiety, and infection of a single pathogenic cell (e.g., bacterium) can be detected by an amplified signal generated via the indicator moiety. Thus the method may comprise detecting an indicator moiety produced during phage replication, wherein detection of the indicator indicates that the bacterium of interest is present in the sample.

**In** an embodiment, the invention comprises a method for detecting *Listeria spp.* in a sample comprising the steps of: incubating the sample with a cocktail composition comprising at least one recombinant bacteriophage constructed from LMA4 that infects the *Listeria spp.,* wherein the recombinant bacteriophage comprises an indicator gene, and an exogenous promoter controlling transcription of the indicator gene, inserted into a late gene region of the bacteriophage, wherein the indicator gene is not contiguous with a gene encoding a structural bacteriophage protein and does not yield a fusion protein, such that expression of the indicator gene during bacteriophage replication following infection of host bacteria results in production of a soluble indicator protein product; and detecting the indicator protein product, wherein positive detection of the indicator protein product indicates that the *Listeria spp.* is present in the sample. In some embodiments, the amount of indicator moiety detected corresponds to the amount of the bacterium of interest present in the sample.

As described in more detail herein, the methods and systems of the invention may utilize a range of concentrations of parental indicator bacteriophage to infect bacteria present in the sample. In some embodiments the indicator bacteriophage are added to the sample at a concentration sufficient to rapidly find, bind, and infect target bacteria that are present in very low numbers in the sample, such as ten cells. In some embodiments, the phage concentration can be sufficient to find, bind, and infect the target bacteria in less than one hour. In other embodiments, these events can occur in less than two hours, or less than three hours, or less than four hours, following addition of indicator phage to the sample. For example, in certain embodiments, the bacteriophage concentration for the incubating step is greater than 1 x 10⁵ PFU/mL, greater than 1 x 10⁶ PFU/mL, or greater than 1 x 10⁷ PFU/mL.

In certain embodiments, the recombinant infectious agent may be purified so as to be free of any residual indicator protein that may be generated upon production of the infectious agent stock. Thus, in certain embodiments, the recombinant bacteriophage may be purified using cesium chloride isopycnic density gradient centrifugation prior to incubation with the sample. When the infectious agent is a bacteriophage, this purification may have the added benefit of removing bacteriophage that do not have DNA (i.e., empty phage or "ghosts").

In some embodiments of the methods of the invention, the microorganism may be detected without any isolation or purification of the microorganisms from a sample. For example, in certain embodiments, a sample containing one or a few microorganisms of interest may be applied directly to an assay container such as a spin column, a microtiter well, or a filter and the assay is conducted in that assay container. Various embodiments of such assays are disclosed herein.

Aliquots of a test sample may be distributed directly into wells of a multi-well plate, indicator phage may be added, and after a period of time sufficient for infection, a lysis buffer may be added as well as a substrate for the indicator moiety (e.g., luciferase substrate for a luciferase indicator) and assayed for detection of the indicator signal. Some embodiments of the method can be performed on filter plates. Some embodiments of the method can be performed with or without concentration of the sample before infection with indicator phage.

For example, in many embodiments, multi-well plates are used to conduct the assays. The choice of plates (or any other container in which detecting may be performed) may affect the detecting step. For example, some plates may include a colored or white background, which may affect the detection of light emissions. Generally speaking, white plates have higher sensitivity but also yield a higher background signal. Other colors of plates may generate lower background signal but also have a slightly lower sensitivity. Additionally, one reason for background signal is the leakage of light from one well to another, adjacent well. There are some plates that have white wells but the rest of the plate is black. This allows for a high signal inside the well but prevents well-to-well light leakage and thus may decrease background. Thus the choice of plate or other assay vessel may influence the sensitivity and background signal for the assay.

Methods of the invention may comprise various other steps to increase sensitivity. For example, as discussed in more detail herein, the method may comprise a step for washing the captured and infected bacterium, after adding the bacteriophage but before incubating, to remove excess parental bacteriophage and/or luciferase or other reporter protein contaminating the bacteriophage preparation.

In some embodiments, detection of the microorganism of interest may be completed without the need for culturing the sample as a way to increase the population of the microorganisms. For example, in certain embodiments the total time required for detection is less than 28.0 hours, 27.0 hours, 26.0 hours, 25.0 hours, 24.0 hours, 23.0 hours, 22.0 hours, 21.0 hours, 20.0 hours, 19.0 hours, 18.0 hours, 17.0 hours, 16.0 hours, 15.0 hours, 14.0 hours, 13.0 hours, 12.0 hours, 11.0 hours, 10.0 hours, 9.0 hours, 8.0 hours, 7.0 hours, 6.0 hours, 5.0 hours, 4.0 hours, 3.0 hours, 2.5 hours, 2.0 hours, 1.5 hours, or less than 1.0 hour. Minimizing time to result is critical in food and environmental testing for pathogens.

In contrast to assays known in the art, the method of the invention can detect individual microorganisms. Thus, in certain embodiments, the method may detect as few as 10 cells of the microorganism present in a sample. For example, in certain embodiments, the recombinant bacteriophage is highly specific for *Listeria spp.* In an embodiment, the recombinant bacteriophage can distinguish *Listeria spp.* in the presence of other types of bacteria. In certain embodiments, the recombinant bacteriophage can be used to detect a single bacterium of the specific type in the sample. In certain embodiments, the recombinant bacteriophage detects as few as 2, 3, 4, 5, 6, 7, 8, 9, 10, 15, 20, 30, 40, 50, 60, 70, 80, 90, or 100 of the specific bacteria in the sample.

Thus, aspects of the present invention provide methods for detection of microorganisms in a test sample via an indicator moiety. In embodiments, the indicator moiety may be associated with an infectious agent, an indicator bacteriophage. The indicator moiety may react with a substrate to emit a detectable signal or may emit an intrinsic signal (e.g., fluorescent protein). In some embodiments, the detection sensitivity can reveal the presence of as few as 50, 40, 30, 20, 10, 5, or 2 cells of the microorganism of interest in a test sample. In some embodiments, even a single cell of the microorganism of interest may yield a detectable signal. In embodiments of the invention, the cocktail composition comprises at least one recombinant bacteriophage constructed from LMA4. In some embodiments, further bacteriophage are a Pecentumvirus, Tequatravirus, Homburgvirus, or Kuttervirus bacteriophage. In some embodiments, the further recombinant bacteriophage is derived from *Listeria-specific* bacteriophage. In certain embodiments, a *recombinant Listeria-specific* bacteriophage is highly specific for *Listeria spp.*

In some embodiments, the indicator moiety encoded by the infectious agent may be detectable during or after replication of the infectious agent. Many different types of detectable biomolecules suitable for use as indicator moieties are known in the art, and many are commercially available. In some embodiments the indicator phage comprises an enzyme, which serves as the indicator moiety. In some embodiments, the genome of the indicator phage is modified to encode a soluble protein. In some embodiments, the indicator phage encodes a detectable enzyme. The indicator may emit light and/or may be detectable by a color change in an added substrate. Various appropriate enzymes are commercially available, such as alkaline phosphatase (AP), horseradish peroxidase (HRP), or luciferase (Luc). In some embodiments, these enzymes may serve as the indicator moiety. In some embodiments, Firefly luciferase is the indicator moiety. In some embodiments, *Oplophorus* luciferase is the indicator moiety. In some embodiments, NANOLUC^{®} is the indicator moiety. Other engineered luciferases or other enzymes that generate detectable signals may also be appropriate indicator moieties.

Thus, in embodiments, the recombinant bacteriophage of the methods, systems or kits is prepared from wild-type *Listeria-specific* bacteriophage LMA4. In some embodiments, the indicator gene encodes a protein that emits an intrinsic signal, such as a fluorescent protein (e.g., green fluorescent protein or others). The indicator may emit light and/or may be detectable by a color change. In some embodiments, the indicator gene encodes an enzyme (e.g., luciferase) that interacts with a substrate to generate signal. In some embodiments, the indicator gene is a luciferase gene. In some embodiments, the luciferase gene is one of *Oplophorus* luciferase, Firefly luciferase, Renilla luciferase, External Gaussia luciferase, Lucia luciferase, or an engineered luciferase such as NANOLUC^{®}, Rluc8.6-535, or Orange Nano-lantern.

Detecting the indicator may include detecting emissions of light. In some embodiments, a luminometer may be used to detect the reaction of indicator (e.g., luciferase) with a substrate. The detection of RLU can be achieved with a luminometer, or other machines or devices may also be used. For example, a spectrophotometer, CCD camera, or CMOS camera may detect color changes and other light emissions. Absolute RLU are important for detection, but the signal to background ratio also needs to be high (e.g., > 2.0, > 2.5, or > 3.0) in order for single cells or low numbers of cells to be detected reliably.

In some embodiments, the indicator phage is genetically engineered to contain the gene for an enzyme, such as a luciferase, which is only produced upon infection of bacteria that the phage specifically recognizes and infects. In some embodiments, the indicator moiety is expressed late in the viral life cycle. In some embodiments, as described herein, the indicator is a soluble protein (e.g., soluble luciferase) and is not fused with a phage structural protein that limits its copy number.

Thus in some embodiments utilizing indicator phage, the invention comprises a method for detecting a microorganism of interest comprising the steps of capturing at least one sample bacterium; incubating the at least one bacterium with a plurality of indicator phage; allowing time for infection and replication to generate progeny phage and express soluble indicator moiety; and detecting the progeny phage, or preferably the indicator, wherein detection of the indicator demonstrates that the bacterium is present in the sample.

For example, in some embodiments the test sample bacterium may be captured by binding to the surface of a plate, or by filtering the sample through a bacteriological filter (e.g., 0.45 µm pore size spin filter or plate filter). In an embodiment, the infectious agent (e.g., indicator phage) is added in a minimal volume to the captured sample directly on the filter. In an embodiment, the microorganism captured on the filter or plate surface is subsequently washed one or more times to remove excess unbound infectious agent. In an embodiment, a medium (e.g., Luria-Bertani (LB) Broth, Buffered Peptone Water (BPW) or Tryptic Soy Broth or Tryptone Soy Broth (TSB), Brain Heart Infusion (BHI) Buffered Listeria Enrichment Broth (BLEB) University of Vermont (UVM) Broth, or Fraser Broth) may be added for further incubation time, to allow replication of bacterial cells and phage and high-level expression of the gene encoding the indicator moiety. However, a surprising aspect of some embodiments of testing assays is that the incubation step with indicator phage only needs to be long enough for a single phage life cycle. The amplification power of using bacteriophage was previously thought to require more time, such that the phage would replicate for several cycles. A single replication cycle of indicator phage can be sufficient to facilitate sensitive and rapid detection according to some embodiments of the present invention.

In some embodiments, aliquots of a test sample comprising bacteria may be applied to a spin column and after infection with a recombinant bacteriophage and an optional washing to remove any excess bacteriophage, the amount of soluble indicator detected will be proportional to the amount of bacteriophage that are produced by infected bacteria.

Soluble indicator (e.g., luciferase) released into the surrounding liquid upon lysis of the bacteria may then be measured and quantified. In an embodiment, the solution is spun through the filter, and the filtrate collected for assay in a new receptacle (e.g., in a luminometer) following addition of a substrate for the indicator enzyme (e.g., luciferase substrate). Alternatively, the indicator signal may be measured directly on the filter.

In various embodiments, the purified parental indicator phage does not comprise the detectable indicator itself, because the parental phage can be purified before it is used for incubation with a test sample. Expression of late (Class III) genes occurs late in the viral life cycle. In some embodiments of the present invention, parental phage may be purified to exclude any existing indicator protein (e.g., luciferase). In some embodiments, expression of the indicator gene during bacteriophage replication following infection of host bacteria results in a soluble indicator protein product. Thus, in many embodiments, it is not necessary to separate parental from progeny phage prior to the detecting step. In an embodiment, the microorganism is a bacterium and the indicator phage is a bacteriophage. In an embodiment, the indicator moiety is soluble luciferase, which is released upon lysis of the host microorganism.

Thus, in an alternate embodiment, the indicator substrate (e.g., luciferase substrate) may be incubated with the portion of the sample that remains on a filter or bound to a plate surface. Accordingly, in some embodiments the solid support is a 96-well filter plate (or regular 96-well plate), and the substrate reaction may be detected by placing the plate directly in the luminometer.

For example, in an embodiment, the invention may comprise a method for detecting *Listeria spp.* comprising the steps of: infecting cells captured on a 96-well filter plate with a plurality of parental indicator phage capable of expressing luciferase upon infection; washing excess phage away; adding BHI broth and allowing time for phage to replicate and lyse the specific *Listeria spp.* target (e.g., 60-240 minutes); and detecting the indicator luciferase by adding luciferase substrate and measuring luciferase activity directly in the 96-well plate, wherein detection of luciferase activity indicates that the *Listeria spp.* is present in the sample.

In another embodiment, the invention may comprise a method for detecting *Listeria spp.* comprising the steps of: infecting cells in liquid solution or suspension in a 96-well plate with a plurality of parental indicator phage capable of expressing luciferase upon infection; allowing time for phage to replicate and lyse the specific *Listeria spp.* target (e.g., 60-240 minutes); and detecting the indicator luciferase by adding luciferase substrate and measuring luciferase activity directly in the 96-well plate, wherein detection of luciferase activity indicates that the *Listeria spp.* is present in the sample. In such an embodiment no capturing step is necessary. In some embodiments, the liquid solution or suspension may be a consumable test sample, such as a vegetable wash. In some embodiments, the liquid solution or suspension may be a vegetable wash fortified with concentrated Luria-Bertani (LB) Broth, Buffered Peptone Water (BPW) or Tryptic Soy Broth or Tryptone Soy Broth (TSB), Brain Heart Infusion (BHI) Buffered Listeria Enrichment Broth (BLEB) University of Vermont (UVM) Broth, or Fraser Broth. In some embodiments, the liquid solution or suspension may be bacteria diluted in BHI Broth.

In some embodiments, lysis of the bacterium may occur before, during, or after the detection step. Experiments suggest that infected unlysed cells may be detectable upon addition of luciferase substrate in some embodiments. Presumably, luciferase may exit cells and/or luciferase substrate may enter cells without complete cell lysis. Thus, for embodiments utilizing the spin filter system, where only luciferase released into the lysate (and not luciferase still inside intact bacteria) is analyzed in the luminometer, lysis is required for detection. However, for embodiments utilizing filter plates or 96-well plates with sample in solution or suspension, where the original plate full of intact and lysed cells is directly assayed in the luminometer, lysis is not necessary for detection.

In some embodiments, the reaction of indicator moiety (e.g., luciferase) with substrate may continue for 60 minutes or more, and detection at various time points may be desirable for optimizing sensitivity. For example, in embodiments using 96-well filter plates as the solid support and luciferase as the indicator, luminometer readings may be taken initially and at 10- or 15-minute intervals until the reaction is completed.

Surprisingly, high concentrations of phage utilized for infecting test samples have successfully achieved detection of very low numbers of a target microorganism in a very short timeframe. The incubation of phage with a test sample in some embodiments need only be long enough for a single phage life cycle. In some embodiments, the bacteriophage concentration for this incubating step is greater than 7 x 10⁶, 8 x 10⁶, 9 x 10⁶, 1.0 x 10⁷, 1.1 x 10⁷, 1.2 x 10⁷, 1.3 x 10⁷, 1.4 x 10⁷, 1.5 x 10⁷, 1.6 x 10⁷, 1.7 x 10⁷, 1.8 x 10⁷, 1.9 x 10⁷, 2.0 x 10⁷, 3.0 x 10⁷, 4.0 x 10⁷, 5.0 x 10⁷, 6.0 x 10⁷, 7.0 x 10⁷, 8.0 x 10⁷, 9.0 x 10⁷, or 1.0 x 10⁸ PFU/mL.

Success with such high concentrations of phage is surprising because the large numbers of phage were previously associated with "lysis from without," which killed target cells and thereby prevented generation of useful signal from earlier phage assays. It is possible that the clean-up of prepared phage stocks described herein helps to alleviate this problem (e.g., clean-up by cesium chloride isopycnic density gradient ultracentrifugation), because in addition to removing any contaminating luciferase associated with the phage, this clean-up may also remove ghost particles (particles that have lost DNA). The ghost particles can lyse bacterial cells via "lysis from without," killing the cells prematurely and thereby preventing generation of indicator signal. Electron microscopy demonstrates that a crude phage lysate (i.e., before cesium chloride clean-up) may have greater than 50% ghosts. These ghost particles may contribute to premature death of the microorganism through the action of many phage particles puncturing the cell membrane. Thus ghost particles may have contributed to previous problems where high PFU concentrations were reported to be detrimental. Moreover, a very clean phage prep allows the assay to be performed with no wash steps, which makes the assay possible to perform without an initial concentration step. Some embodiments do include an initial concentration step, and in some embodiments this concentration step allows a shorter enrichment incubation time.

Some embodiments of testing methods may further include confirmatory assays. A variety of assays are known in the art for confirming an initial result, usually at a later point in time. For example, the samples can be cultured (e.g., selective chromogenic plating), and PCR can be utilized to confirm the presence of the microbial DNA, or other confirmatory assays can be used to confirm the initial result.

In certain embodiments, the methods of the present invention combine the use of a binding agent (e.g., antibody) to purify and/or concentrate a microorganism of interest such as *Listeria* spp. from the sample in addition to detection with an infectious agent. For example, in certain embodiments, the present invention comprises a method for detecting a microorganism of interest in a sample comprising the steps of: capturing the microorganism from the sample on a prior support using a capture antibody specific to the microorganism of interest such as *Listeria spp*.; incubating the sample with a recombinant bacteriophage that infects *Listeria spp.* wherein the recombinant bacteriophage comprises an indicator gene inserted into a late gene region of the bacteriophage such that expression of the indicator gene during bacteriophage replication following infection of host bacteria results in a soluble indicator protein product; and detecting the indicator protein product, wherein positive detection of the indicator protein product indicates that *Listeria spp.* is present in the sample.

In some embodiments synthetic phage are designed to optimize desirable traits for use in pathogen detection assays. In some embodiments bioinformatics and previous analyses of genetic modifications are employed to optimize desirable traits. For example, in some embodiments, the genes encoding phage tail proteins can be optimized to recognize and bind to particular species of bacteria. In other embodiments the genes encoding phage tail proteins can be optimized to recognize and bind to an entire genus of bacteria, or a particular group of species within a genus. In this way, the phage can be optimized to detect broader or narrower groups of pathogens. In some embodiments, the synthetic phage may be designed to improve expression of the reporter gene. Additionally and/or alternatively, in some instances, the synthetic phage may be designed to increase the burst size of the phage to improve detection.

In some embodiments, the stability of the phage may be optimized to improve shelf-life. For example, enzybiotic solubility may be increased in order to increase subsequent phage stability. Additionally and/or alternatively phage thermostability may be optimized. Thermostable phage better preserve functional activity during storage thereby increasing shelf-life. Thus, in some embodiments, the thermostability and/or pH tolerance may be optimized.

In some embodiments the genetically modified phage or the synthetically derived phage comprises a detectable indicator. In some embodiments the indicator is a luciferase. In some embodiments the phage genome comprises an indicator gene (e.g., a luciferase gene or another gene encoding a detectable indicator).

### Systems and Kits of the Invention

In some embodiments, the invention comprises systems (e.g., automated systems or kits) comprising components for performing the methods defined in the appended claims. In some embodiments, indicator phage are comprised in systems or kits according to the invention. Methods described herein may also utilize such indicator phage systems or kits. Some embodiments described herein are particularly suitable for automation and/or kits, given the minimal amount of reagents and materials required to perform the methods. In certain embodiments, each of the components of a kit may comprise a self-contained unit that is deliverable from a first site to a second site.

In some embodiments, the invention comprises systems or kits for rapid detection of a *Listeria spp.* of interest in a sample. The systems or kits may in certain embodiments comprise a component for incubating the sample with the cocktail composition comprises at least one recombinant bacteriophage constructed from LMA4 , the recombinant bacteriophage comprising an indicator gene, and an exogenous promoter controlling transcription of the indicator gene, inserted into a late gene region of a bacteriophage genome, wherein the indicator gene is not contiguous with a gene encoding a structural bacteriophage protein and does not yield a fusion protein, and wherein expression of the indicator gene results in an indicator protein product, and wherein the recombinant bacteriophage specifically infects *Listeria spp..* Some systems further comprise a component for capturing the microorganism of interest on a solid support.

In other embodiments, the invention comprises a method, system, or kit for rapid detection of a microorganism of interest in a sample, comprising a cocktail composition comprising at least one recombinant bacteriophage constructed from LMA4 , the recombinant bacteriophage comprising an indicator gene, and an exogenous promoter controlling transcription of the indicator gene, inserted into a late gene region of a bacteriophage genome, wherein the indicator gene is not contiguous with a gene encoding a structural bacteriophage protein and does not yield a fusion protein, and wherein expression of the indicator gene results in an indicator protein product, and wherein the recombinant bacteriophage specifically infects *Listeria spp*.In an embodiment, the recombinant bacteriophage can distinguish *Listeria spp.* in the presence of other types of bacteria. In certain embodiments, a system or kit detects as few as 1, 10, 15, 20, 30, 40, 50, 60, 70, 80, 90, or 100 specific bacteria in the sample.

In certain embodiments, the systems and/or kits may further comprise a component for washing the captured microorganism sample. Additionally or alternatively, the systems and/or kits may further comprise a component for determining amount of the indicator moiety, wherein the amount of indicator moiety detected corresponds to the amount of microorganism in the sample. For example, in certain embodiments, the system or kit may comprise a luminometer or other device for measuring a luciferase enzyme activity.

In some systems and/or kits, the same component may be used for multiple steps. In some systems and/or kits, the steps are automated or controlled by the user via computer input and/or wherein a liquid-handling robot performs at least one step.

Thus in certain embodiments, the invention may comprise a system or kit for rapid detection *Listeria spp.* in a according to the appended claims.

The infectious agent may comprise *Listeria-specific* NANOLUC^{®} bacteriophage carrying the NANOLUC^{®} indicator gene. The systems or kits may comprise a variety of components for detection of progeny infectious agents. For example, the progeny infectious agent ( bacteriophage) comprise an indicator moiety. In an embodiment the indicator moiety in the progeny infectious agent ( bacteriophage) may be a detectable moiety that is expressed during replication, such as a soluble luciferase protein.

### Computer Systems and Computer Readable Media which are not part of the invention

The system, as described in the present technique or any of its components, may be embodied in the form of a computer system. Typical examples of a computer system include a general-purpose computer, a programmed microprocessor, a microcontroller, a peripheral integrated circuit element, and other devices or arrangements of devices that are capable of implementing the steps that constitute the method of the present technique.

A computer system may comprise a computer, an input device, a display unit, and/or the Internet. The computer may further comprise a microprocessor. The microprocessor may be connected to a communication bus. The computer may also include a memory. The memory may include random access memory (RAM) and read only memory (ROM). The computer system may further comprise a storage device. The storage device can be a hard disk drive or a removable storage drive such as a floppy disk drive, optical disk drive, etc. The storage device can also be other similar means for loading computer programs or other instructions into the computer system. The computer system may also include a communication unit. The communication unit allows the computer to connect to other databases and the Internet through an I/O interface. The communication unit allows the transfer to, as well as reception of data from, other databases. The communication unit may include a modem, an Ethernet card, or any similar device which enables the computer system to connect to databases and networks such as LAN, MAN, WAN and the Internet. The computer system thus may facilitate inputs from a user through input device, accessible to the system through I/O interface.

A computing device typically will include an operating system that provides executable program instructions for the general administration and operation of that computing device, and typically will include a computer-readable storage medium (e.g., a hard disk, random access memory, read only memory, etc.) storing instructions that, when executed by a processor of the server, allow the computing device to perform its intended functions. Suitable implementations for the operating system and general functionality of the computing device are known or commercially available, and are readily implemented by persons having ordinary skill in the art, particularly in light of the disclosure herein.

The computer system executes a set of instructions that are stored in one or more storage elements, in order to process input data. The storage elements may also hold data or other information as desired. The storage element may be in the form of an information source or a physical memory element present in the processing machine.

The environment can include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers, or other network devices may be stored locally and/or remotely, as appropriate. Where a system includes computing devices, each such device can include hardware elements that may be electrically coupled via a bus, the elements including, for example, at least one central processing unit (CPU), at least one input device (e.g., a mouse, keyboard, controller, touch screen, or keypad), and at least one output device (e.g., a display device, printer, or speaker). Such a system may also include one or more storage devices, such as disk drives, optical storage devices, and solid-state storage devices such as random access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.), and working memory as described above. The computer-readable storage media reader can be connected with, or configured to receive, a computer-readable storage medium, representing remote, local, fixed, and/or removable storage devices as well as storage media for temporarily and/or more permanently containing, storing, transmitting, and retrieving computer-readable information. The system and various devices also typically will include a number of software applications, modules, services, or other elements located within at least one working memory device, including an operating system and application programs, such as a client application or Web browser. It should be appreciated that alternate embodiments may have numerous variations from that described above. For example, customized hardware might also be used and/or particular elements might be implemented in hardware, software (including portable software, such as applets), or both. Further, connection to other computing devices such as network input/output devices may be employed.

Non-transient storage media and computer readable media for containing code, or portions of code, can include any appropriate media known or used in the art, including storage media and communication media, such as but not limited to volatile and non-volatile, removable and non-removable media implemented in any method or technology for storage and/or transmission of information such as computer readable instructions, data structures, program modules, or other data, including RAM, ROM, EEPROM, flash memory or other memory technology, CD-ROM, digital versatile disk (DVD) or other optical storage, magnetic cassettes, magnetic tape, magnetic disk storage or other magnetic storage devices, or any other medium which can be used to store the desired information and which can be accessed by the a system device. Based on the disclosure and teachings provided herein, a person of ordinary skill in the art will appreciate other ways and/or methods to implement the various embodiments.

A computer-readable medium may comprise, but is not limited to, an electronic, optical, magnetic, or other storage device capable of providing a processor with computer-readable instructions. Other examples include, but are not limited to, a floppy disk, CD-ROM, DVD, magnetic disk, memory chip, ROM, RAM, SRAM, DRAM, content-addressable memory ("CAM"), DDR, flash memory such as NAND flash or NOR flash, an ASIC, a configured processor, optical storage, magnetic tape or other magnetic storage, or any other medium from which a computer processor can read instructions. In one embodiment, the computing device may comprise a single type of computer-readable medium such as random access memory (RAM). In other embodiments, the computing device may comprise two or more types of computer-readable medium such as random access memory (RAM), a disk drive, and cache. The computing device may be in communication with one or more external computer-readable mediums such as an external hard disk drive or an external DVD or Blu-Ray drive.

As discussed above, the embodiment comprises a processor which is configured to execute computer-executable program instructions and/or to access information stored in memory. The instructions may comprise processor-specific instructions generated by a compiler and/or an interpreter from code written in any suitable computer-programming language including, for example, C, C++, C#, Visual Basic, Java, Python, Perl, JavaScript, and ActionScript (Adobe Systems, Mountain View, Calif.). In an embodiment, the computing device comprises a single processor. In other embodiments, the device comprises two or more processors. Such processors may comprise a microprocessor, a digital signal processor (DSP), an application-specific integrated circuit (ASIC), field programmable gate arrays (FPGAs), and state machines. Such processors may further comprise programmable electronic devices such as PLCs, programmable interrupt controllers (PICs), programmable logic devices (PLDs), programmable read-only memories (PROMs), electronically programmable read-only memories (EPROMs or EEPROMs), or other similar devices.

The computing device comprises a network interface. In some embodiments, the network interface is configured for communicating via wired or wireless communication links. For example, the network interface may allow for communication over networks via Ethernet, IEEE 802.11 (Wi-Fi), 802.16 (Wi-Max), Bluetooth, infrared, etc. As another example, network interface may allow for communication over networks such as CDMA, GSM, UMTS, or other cellular communication networks. In some embodiments, the network interface may allow for point-to-point connections with another device, such as via the Universal Serial Bus (USB), 1394 FireWire, serial or parallel connections, or similar interfaces. Some embodiments of suitable computing devices may comprise two or more network interfaces for communication over one or more networks. In some embodiments, the computing device may include a data store in addition to or in place of a network interface.

Some embodiments of suitable computing devices may comprise or be in communication with a number of external or internal devices such as a mouse, a CD-ROM, DVD, a keyboard, a display, audio speakers, one or more microphones, or any other input or output devices. For example, the computing device may be in communication with various user interface devices and a display. The display may use any suitable technology including, but not limited to, LCD, LED, CRT, and the like.

The set of instructions for execution by the computer system may include various commands that instruct the processing machine to perform specific tasks such as the steps that constitute the method of the present technique. The set of instructions may be in the form of a software program. Further, the software may be in the form of a collection of separate programs, a program module with a larger program or a portion of a program module, as in the present technique. The software may also include modular programming in the form of object-oriented programming. The processing of input data by the processing machine may be in response to user commands, results of previous processing, or a request made by another processing machine.

### EXAMPLES

Results depicted in the following examples demonstrate detection of a low number of cells, as few as 1 *Listeria* bacteria, in a shortened time to results.

### Example 1. Creation and Isolation of Indicator Phage from Listeria-Specific Bacteriophage

Indicator Phage *Listeria-specific* LMA4.NANOLUC, LMA8.NANOLUC, and other *Listeria* bacteriophages were created through homologous recombination using procedures as previously described. See Figures 1-3 which depict and describe recombinant *Listeria* phages derived from LMA4 and LMA8.

The genomic sequences of these phage were obtained through whole genome sequencing using the Illumina MiSeq system with de novo sequence assembly. Based on previously known and annotated genomes of related phage, the late gene regions and Major Capsid Protein genes were located on the new phage genomes. Plasmids were designed and synthesized to insert NANOLUC^{®} along with the appropriate late gene promoter and ribosomal binding site, flanked by approximately 200-500 bp of matching phage sequence to promote homologous recombination.

Target bacteria were transformed with the Homologous Recombination Plasmids under appropriate antibiotic selection and infected with their respective wild-type phage to allow for homologous recombination with the plasmid. Following homologous recombination to generate the recombinant bacteriophage genomes, a series of titer and enrichment steps was used to isolate specific recombinant bacteriophages that express NANOLUC^{®} as previously described.

Finally, large-scale production was performed to obtain high titer stocks appropriate for use in the *Listeria spp.* detection assays. Cesium chloride isopycnic density gradient centrifugation may be used to separate phage particles from contaminating luciferase protein to reduce background. In other embodiments, sucrose isopycnic density gradient centrifugation may be used to separate phage particles from contaminating luciferase protein to reduce background.

### Example 2. Inoculated Sponge Sample-Sponge Assay for Listeria

EZ Reach polyurethabe sponge samplers were pre-wetted with Dey/Engley Broth and spiked with < 1 CFU of *Listeria monocytogenes,* which was diluted from an overnight culture or with 100 CFU challenge bacteria (*Cronobacter sakazakii*)*.*

The handle of the sponge was broken off and the sponge was placed into medium in a bag. Buffered Listeria Enrichment Broth (BLEB) (Remel) medium (10 or 90 mL) was added to cover as much of the sponge as possible. The sponge was then gently massaged to release bacteria into the medium and enrichment followed at 35 °C for 16-18 hours or 24 hours. After enrichment, sponges were gently massaged/squeezed to remove the liquid and were then moved away from the medium in the bag. The bag was then gently massaged to mix the contents. 150 µL aliquots were transferred to a 96-well plate. The sponge was then placed into the medium for further enrichment at 35 °C, if necessary.

Sponge samples were tested with *Listeria* phage cocktail following 1-hour and 4-hour infection. Briefly, phage reagent (10 µL) was added to samples and the samples incubated at 30 °C for 1 hour or 4 hours. Finally, 65 µL of Luciferase Master Mix reagent was added to each well and gently mixed by pipetting up and down. Samples were read (i.e., luminescence detected) on a luminometer (GloMax96) instrument 3 minutes after substrate addition. Sponges/swabs were placed back into the bag/tube and enrichment continued at 35 °C for a total of 24 hours. Optionally, aliquots may be taken and further enriched and tested again.

Results are shown in **Figures 5A and 5B****.** A signal to background ratio (S/B) greater than 3 was considered positive. The background level was determined to be 100 RLU based on prior phage characterizations. These experiments indicate that the Listeria Phage Assay can detect a 1 CFU spike of *L. monocytogenes* ATCC 19115 following 16-18 hours of enrichment and 1 hour of infection. **Figure 5A** (10 mL medium) shows that sponge samples 1 and 4 were negative for detection of *Listeria* (i.e., S/B < 3.0). Sponge samples 2, 3, 5, and the 10 CFU control were all positive for all enrichment and infection times. **Figure 5B** (90 mL medium) shows that sponge 1 was negative for detection of *Listeria* while all other samples had positive detection. These data indicate that sponges with 10 mL of added medium generated a better signal with higher relative S/B than samples with 90 mL added medium.

Thus the experiment demonstrates it is possible to detect 1 CFU spike from an overnight culture with all the conditions tried (i.e., 16-18 hr enrichment - 1 hr infection, 16-18 hr enrichment - 4 hr infection, 24 hr enrichment -1 hr infection, and 24 hr enrichment - 4 hr infection.

### Example 3. Environmental Surface Sample-Sponge Assay for Listeria

Stainless steel surfaces were inoculated with the indicated number of cells in medium. Cells were allowed dry onto the surface and kept at room temperature for 18-24 hours before being swabbed with EZ Reach polyurethane sponge samplers were pre wet with Letheen medium (World BioProducts). *Listeria monocytogenes* 19115 was used as the target and *Staphylococcus aureus* 12600 was used as the challenge strain.

The handle of the sponge was broken off and the sponge was placed back into medium in the bag. Buffered Listeria Enrichment Broth (BLEB) (Remel) medium (20 mL) was added to cover as much of the sponge in medium as possible. The sponge was gently massaged to release bacteria into the medium and enrichment followed at 35 °C for 20 hours. After enrichment, sponges were gently massaged, squeezed to remove the liquid, and then moved away from the medium in the bag. The bag was gently massaged to mix the contents. 150 µL aliquots were transferred to a 96-well plate. The sponge was replaced into the medium and incubated at 35 °C if further enrichment was necessary.

Sponge samples were tested with Listeria phage cocktail following 1-hour and 4-hour infection. Briefly, phage reagent (10 µL) was added to samples and incubated at 30 °C for 4 hours. Finally, 65 µL of Luciferase Master Mix reagent was added to each well and gently mixed by pipetting up and down. Samples were read (i.e., luminescence detected) on a GloMax96 instrument 3 minutes after substrate addition.

Phage reagent (10 µL) was added to samples and incubated at 30 °C for 4 hours. Finally 65 µL of Luciferase Master Mix reagent was added to each well and gently mixed by pipetting up and down. Samples were read (i.e., luminescence detected) on a GloMax96 instrument 3 minutes (180 seconds) after substrate addition.

**Figure 6** shows the RLU generated from the stainless steel surface swabs. Samples generating RLU >300 were considered positive. These data show that the Listeria Phage Assay can detect a 100 CFU surface inoculation of *L. monocytogenes* with a 20-hour enrichment and 4 hours of infection in the presence of a non-*Listeria* bacteria present at a 10-fold greater CFU level than the target *Listeria* bacteria. Sample 2 and the negative control were negative for detection of *L. monocytogenes.* All other samples, including the positive control, were positive. Compared to the spiked sponge experiments, the swabs of stainless steel required longer enrichment period and a higher CFU level for positive detection. This can be attributed to several variables, including increased injury to surface inoculated cells as compared to those from an overnight culture. Surface inoculated cells typically have significant loss of viability. Also, recovery of cells form a surface with sponges can be highly variable.

## Claims

1. A cocktail composition comprising at least one recombinant bacteriophage constructed from LMA4,
comprising:
(i) an indicator gene, and
(ii) an exogenous promoter controlling transcription of the indicator gene,
inserted into a late gene region of the bacteriophage genome;
wherein the indicator gene is not contiguous with a gene encoding a structural bacteriophage protein and does not yield a fusion protein,
and wherein expression of the indicator gene results in an indicator protein product,
and wherein the recombinant bacteriophage specifically infects *Listeria spp.*

2. A method for detecting *Listeria spp.* in a sample comprising:
incubating the sample with the cocktail composition according to claim 1; and
detectingthe indicator protein product produced by the recombinant bacteriophage, wherein positive detection of the indicator protein product indicates that *Listeria spp.* Is present in the sample.

3. The method of claim 2, wherein the sample is a food, environmental, water, or commercial sample.

4. The method of claims 2 to 3, wherein the method detects as few as 10, 9, 8, 7, 6, 5, 4, 3, 2, or a single bacterium in a sample of a standard size for the food safety industry.

5. The method of claim 3, wherein the food sample comprises meat, fish, vegetables, eggs, dairy products, dried food products, or powdered infant formula.

6. The method of any one of claims 2 to 5, wherein the sample is first incubated in conditions favoring growth for an enrichment period of less than 24 hours, 23 hours, 22 hours, 21 hours, 20 hours, 19 hours, 18 hours, 17 hours, 16 hours, 15 hours, 14 hours, 13 hours, 12 hours, 11 hours, 10 hours, 9 hours, 8 hours, 7 hours, 6 hours, 5 hours, 4 hours, 3 hours, or 2 hours.

7. The method of any one of claims 2 to 6, wherein the total time to results is less than 28 hours, 27 hours, 26 hours, 25 hours, 24 hours, 23 hours, 22 hours, 21 hours, 20 hours, 19 hours, 18 hours, 17 hours, 16 hours, 15 hours, 14 hours, 13 hours, 12 hours, 11 hours, 10 hours, 9 hours 8 hours, 7 hours, 6 hours, 5 hours, 4 hours, 3 hours, or 2 hours.

8. The method of any one of claims 2 to 7, wherein the ratio of signal to background generated by detecting the indicator is at least 2.0 or at least 2.5 or at least 3.0.

9. A kit for detecting *Listeria spp.* comprising the cocktail composition according to claim 1.

10. The kit of claim 9, further comprising a substrate for reacting with an indicator to detect the soluble protein product expressed by the recombinant bacteriophage.

11. A system for detecting *Listeria spp.* comprising the cocktail composition according to claim 1.

## Patentansprüche

1. Cocktailzusammensetzung, umfassend mindestens einen rekombinanten Bakteriophagen, der aus LMA4 konstruiert wird, umfassend:
(i) ein Indikatorgen und
(ii) einen exogenen Promotor, der eine Transkription des Indikatorgens steuert, eingefügt in eine späte Genregion des Bakteriophagengenoms;
wobei das Indikatorgen nicht an ein Gen, das für ein strukturelles Bakteriophagenprotein codiert, grenzt und kein Fusionsprotein ergibt,
und wobei Expression des Indikatorgens in einem Indikatorproteinprodukt resultiert,
und wobei der rekombinante Bakteriophage *Listeria spp.* speziell infiziert.

2. Verfahren zum Erkennen von *Listeria spp.* in einer Probe, umfassend:
Inkubieren der Probe mit der Cocktailzusammensetzung nach Anspruch 1; und
Erkennen des Indikatorproteinprodukts, das durch den rekombinanten Bakteriophagen produziert wird, wobei eine positive Erkennung des Indikatorproteinprodukts anzeigt, dass *Listeria spp.* in der Probe vorhanden ist;

3. Verfahren nach Anspruch 2, wobei die Probe eine Lebensmittel-, Umwelt-, Wasser- oder Handelsprobe ist.

4. Verfahren nach Anspruch 2 bis 3, wobei das Verfahren so wenig wie 10, 9, 8, 7, 6, 5, 4, 3, 2 oder ein einzelnes Bakterium in einer Probe einer Standardgröße für die Lebensmittelsicherheitsindustrie erkennt.

5. Verfahren nach Anspruch 3, wobei die Lebensmittelprobe Fleisch, Fisch, Gemüse, Eier, Milchprodukte, getrocknete Lebensmittelprodukte oder pulverisierte Säuglingsnahrung umfasst.

6. Verfahren nach einem der Ansprüche 2 bis 5, wobei die Probe zuerst unter wachstumsfördernden Bedingungen für einen Anreicherungszeitraum von weniger als 24 Stunden, 23 Stunden, 22 Stunden, 21 Stunden, 20 Stunden, 19 Stunden, 18 Stunden, 17 Stunden, 16 Stunden, 15 Stunden, 14 Stunden, 13 Stunden, 12 Stunden, 11 Stunden, 10 Stunden, 9 Stunden, 8 Stunden, 7 Stunden, 6 Stunden, 5 Stunden, 4 Stunden, 3 Stunden oder 2 Stunden inkubiert wird.

7. Verfahren nach einem der Ansprüche 2 bis 6, wobei die Gesamtzeit zu Resultaten weniger als 28 Stunden, 27 Stunden, 26 Stunden, 25 Stunden, 24 Stunden, 23 Stunden, 22 Stunden, 21 Stunden, 20 Stunden, 19 Stunden, 18 Stunden, 17 Stunden, 16 Stunden, 15 Stunden, 14 Stunden, 13 Stunden, 12 Stunden, 11 Stunden, 10 Stunden, 9 Stunden, 8 Stunden, 7 Stunden, 6 Stunden, 5 Stunden, 4 Stunden, 3 Stunden oder 2 Stunden beträgt.

8. Verfahren nach einem der Ansprüche 2 bis 7, wobei das Verhältnis von Signal zu Hintergrund, das durch das Erkennen des Indikators erzeugt wird, mindestens 2,0 oder mindestens 2,5 oder mindestens 3,0 beträgt.

9. Kit zum Erkennen von *Listeria spp.*, umfassend die Cocktailzusammensetzung nach Anspruch 1.

10. Kit nach Anspruch 9, ferner umfassend ein Substrat zum Reagieren mit einem Indikator, um das lösliche Proteinprodukt, das durch den rekombinanten Bakteriophagen exprimiert wird, zu erkennen.

11. System zum Erkennen von *Listeria spp.*, umfassend die Cocktailzusammensetzung nach Anspruch 1.

## Revendications

1. Composition de cocktail comprenant au moins un bactériophage recombinant construit à partir de LMA4, comprenant :
(i) un gène indicateur, et
(ii) un promoteur exogène contrôlant la transcription du gène indicateur, inséré dans une région du gène tardif du génome de bactériophage ;
dans laquelle le gène indicateur n'est pas contigu à un gène codant pour une protéine structurelle de bactériophage et ne produit pas de protéine de fusion,
et dans laquelle l'expression du gène indicateur donne lieu à un produit protéiné indicateur,
et dans laquelle le bactériophage recombinant infecte spécifiquement la *Listeria spp.*

2. Procédé de détection de *Listeria spp.* dans un échantillon comprenant :
l'incubation de l'échantillon avec la composition de cocktail selon la revendication 1 ; et
la détection du produit protéiné indicateur produit par le bactériophage recombinant, dans lequel la détection positive du produit protéiné indicateur indique que la *Listeria spp.* est présente dans l'échantillon ;

3. Procédé selon la revendication 2, dans lequel l'échantillon est un échantillon alimentaire, environnemental, d'eau ou commercial.

4. Procédé selon les revendications 2 ou 3, dans lequel le procédé détecte aussi peu que 10, 9, 8, 7, 6, 5, 4, 3, 2, ou une seule bactérie dans un échantillon de taille standard pour l'industrie de sécurité alimentaire ;

5. Procédé selon la revendication 3, dans lequel l'échantillon alimentaire comprend de la viande, du poisson, des légumes, des œufs, des produits laitiers, des produits alimentaires séchés ou des préparations en poudre pour nourrissons.

6. Procédé selon l'une quelconque des revendications 2 à 5, dans lequel l'échantillon est d'abord incubé dans des conditions favorisant la croissance pendant une période d'enrichissement inférieure à 24 heures, 23 heures, 22 heures, 21 heures, 20 heures, 19 heures, 18 heures, 17 heures, 16 heures, 15 heures, 14 heures, 13 heures, 12 heures, 11 heures, 10 heures, 9 heures, 8 heures, 7 heures, 6 heures, 5 heures, 4 heures, 3 heures, ou 2 heures.

7. Procédé selon l'une quelconque des revendications 2 à 6, dans lequel la durée totale des résultats est inférieure à 28 heures, 27 heures, 26 heures, 25 heures, 24 heures, 23 heures, 22 heures, 21 heures, 20 heures, 19 heures, 18 heures, 17 heures, 16 heures, 15 heures, 14 heures, 13 heures, 12 heures, 11 heures, 10 heures, 9 heures, 8 heures, 7 heures, 6 heures, 5 heures, 4 heures, 3 heures ou 2 heures.

8. Procédé selon l'une quelconque des revendications 2 à 7, dans lequel le rapport de signal à l'arrière-plan généré par la détection de l'indicateur est d'au moins 2,0 ou d'au moins 2,5 ou d'au moins 3,0.

9. Kit de détection de *Listeria spp.* comprenant la composition de cocktail selon la revendication 1.

10. Kit selon la revendication 9, comprenant en outre un substrat pour réagir avec un indicateur afin de détecter le produit protéiné soluble exprimé par le bactériophage recombinant.

11. Système de détection de *Listeria spp.* comprenant la composition de cocktail selon la revendication 1.
